(19) Europäisches Patentamt
European Patent Office
Office européen des brevets

(11) EP 4 474 372 A1

# (12) EUROPEAN PATENT APPLICATION

(43) Date of publication:
**11.12.2024 Bulletin 2024/50**

(21) Application number: **24186039.4**

(22) Date of filing: **09.12.2021**

(51) International Patent Classification (IPC):
***C07C 1/20*** *(2006.01)* ***C07C 1/24*** *(2006.01)*
***C07C 11/02*** *(2006.01)*

(52) Cooperative Patent Classification (CPC):
(C-Sets available)
**C07C 1/20; C07C 1/24;** Y02P 20/582 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.12.2020 PCT/EP2020/085177**

(62) Document number(s) of the earlier application(s) in accordance with Art. 76 EPC:
**21831295.7 / 4 110 747**

(71) Applicant: **Symrise AG**
**37603 Holzminden (DE)**

(72) Inventors:
- **BUGDAHN, Nikolas**
**37603 Holzminden (DE)**
- **STRUEVER, Frank**
**31789 Hameln (DE)**
- **HEINEMEYER, Heiko**
**37627 Wangelnstedt (DE)**
- **HOPPE, Moritz**
**37671 Hoexter-Luetmarsen (DE)**
- **SIEWERT, Juergen**
**37434 Rollshausen (DE)**
- **LANGE, Sabine**
**37603 Holzminden (DE)**
- **RUSSBUELDT, Bernhard**
**37671 Hoexter (DE)**

(74) Representative: **Global IP Europe Patentanwaltskanzlei**
**Pfarrstraße 14**
**80538 München (DE)**

Remarks:
This application was filed on 02-07-2024 as a divisional application to the application mentioned under INID code 62.

## (54) PROCESS FOR THE EFFICIENT PREPARATION OF (BIO)-ALKANEDIOLS

(57) The present invention relates to a process for the efficient, sustainable and gentle preparation of bio-based alkanediols, especially 1,2-alkanediols, at moderate temperatures based on a recycling of side products and non-reacted educts. The present invention also relates to a composition comprising at least two alkanediols obtainable by said process and to the use of said composition in consumer product formulations. In addition, the present invention relates to consumer products as such comprising the inventive composition.

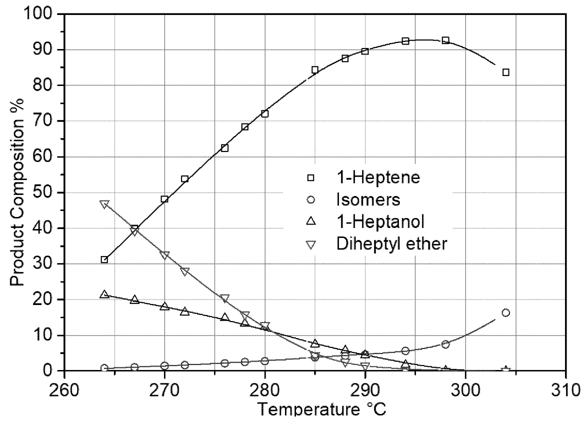


Figure 1A



EP 4 474 372 A1

(52) Cooperative Patent Classification (CPC): (Cont.)

C-Sets
**C07C 1/20, C07C 11/02;**
**C07C 1/24, C07C 11/02**

## Description

### Technical field

The present invention relates to a process for the efficient, sustainable, cost-effective and gentle preparation of (bio-based) alkanediols, in particular 1,2-alkanediols having 5 to 15 carbon atoms. The present invention also relates to a composition comprising at least two alkanediols preferably obtainable by said process and to the use of said composition in consumer product formulations. In addition, the present invention relates to consumer products as such comprising the inventive composition.

### Background Art

It has been recently found that specific alkanediols, and in particular specific 1,2-alkanediols such as 1,2-hexanediol and 1,2-octanediol, can be advantageously added to a broad variety of consumer product formulations as multifunctional actives and during the last years several methods have been developed for the preparation of these substances.

Such alkanediols are usually prepared by hydrolysis of epoxyalkanes based on the epoxidation of olefins. However, often by-products are formed which are causing an unpleasant smell, which is why additional effort was made to improve said processes e.g. by additional purification methods.

For example, EP 1 876 162 A1 describes the preparation of alkanediol compounds having four or more carbon atoms from the corresponding olefins by means of epoxidation and subsequent hydrolysis followed by an additional treatment based on the addition of water and/or an organic solvent to the alkanediol composition and the subsequent removal of the water and/or the organic solvent under reduced pressure for purification and the removal of side products such as esters and dioxanes which might cause disadvantageous smells.

US 6,528,665 B1 proposes the preparation of pure alkanediols prepared from the corresponding epoxyalkanes, based on the epoxidation of olefinic compounds with hydrogen peroxide, subjecting the epoxyalkanes to a complex washing treatment using aqueous washing solutions of inorganic bases and borohydride, and subsequent hydrolyzation.

However, both processes require the use of additional chemicals and solvents as well as energy and generate a considerable amount of waste products. Consequently, these processes are neither ecologically nor economically efficient. In addition, both processes do not refer to the provision of the olefinic starting compounds.

EP 0 257 243 A1 discloses the preparation of low molecular weight vicinal diols by saponification of the corresponding epoxides with water by using specific epoxide to water ratios in the presence of a catalyst. A comparable process comprising reacting a 1-alkene with formic acid and hydrogen peroxide and subsequent saponification is disclosed in EP 0 141 775 A1 and requires several reaction stages connected in series. However, these processes have multiple economic and ecological disadvantages. For example, the diester of 1,2-pentanediol that is formed as an intermediate in the process must be saponified in order to obtain 1,2-pentanediol. If the epoxidation of n-pent-1-ene is carried out, for example, with hydrogen peroxide and formic acid, sodium formate is formed as a coupling product in the subsequent saponification of the diformate of 1,2-pentanediol with sodium hydroxide solution and must be disposed of, thus leading to a high load of organics in the wastewater.

Another way to obtain alkanediols such as 1,2-pentanediol from renewable sources is described in WO 2019/152569 A2. WO 2019/152569 A2 discloses a process for converting an alkene to a 1,2-alkanediol, wherein the alkene is obtained by dehydrating a 1-alcohol in a reactor by heating in the presence of a specifically modified and high-temperature calcinated catalyst. Thereby, the process is based on a single pass of the gaseous reactants over the catalyst at high temperatures of approximately 315 °C. However, this process requires the application of high temperatures and additional steps for the complex catalyst preparation and conversion of the alcohol to the alkene resulting in an economically and ecologically disadvantageous preparation process in conjunction with increased amounts of wastes produced upon catalyst preparation.

Therefore, none of the processes identified above provides for an economically and ecologically efficient and friendly method for the preparation of alkanediols, especially 1,2-alkanediols and the corresponding intermediate olefins. In particular, none of these processes allows for the economically and ecologically efficient preparation of 1,2-heptanediol, 1,2-octanediol and/or 1,2-nonanediol and the corresponding intermediate alkene compounds.

In addition, the desire for more sustainable preparation methods has increased over the past years and there is an ongoing need to improve the preparation of these valuable alkanediol compositions. Consequently, there is a high demand for fully efficient and sustainable preparation methods for these alkanediols as such.

It is therefore an object of the present invention to provide for a more sustainable, i.e., a more ecological and more economical process for the preparation of alkanediols having 5 to 15 carbon atoms using milder reaction conditions while simultaneously allowing for an improved balance of selectivity, yield and purity as well as a reduction of waste products, in particular in the preparation of the intermediate olefin compounds.

Furthermore, there is a need to improve the properties of the final process products in terms of solubility, odour and the like. Consequently, it is another object of the present invention to provide for an improved alkanediol product. Thereby, no by-products should be comprised which negatively influence the physical and chemical properties of the alkanediols such as their colour, their odour or their solubility.

These and other objects and advantages of the present invention will become clear from the following disclosure.

Surprisingly, it has now been found in a first aspect that the process described herein allows for such an improved, sustainable, and simultaneously efficient, preparation of high-purity alkanediols or alkanediol-compositions at moderate reaction conditions, in particular at moderate temperatures, allowing for the efficient preparation of alkanediols or alkanediol-compositions via the preparation of high-purity alkenes or alkene-compositions as intermediates, respectively. In addition, in total less process steps are required, no complex and costly modification of the catalyst is necessary, and the overall costs and waste amounts are considerably reduced compared to the state-of-the-art approaches. Moreover, based on the process described herein, it is possible to prepare alkanediols and/or alkanediol compositions/mixtures having improved properties.

The present invention is specified in the appended claims. The invention itself, and its preferred variants, other objects and advantages, are however also apparent from the following detailed description in conjunction with the accompanying examples and figures.

**Summary of the invention**

In a first aspect, the present invention relates to a more ecological and economically more efficient process for the preparation of high-purity alkanediols or alkanediol-compositions having 5 to 15 carbon atoms, preferably 1,2-alkanediols, comprising the following steps:

a) providing a 1-alcohol having 5 to 15 carbon atoms and/or a dialkylether having 10 to 30 carbon atoms, and a catalyst;

b) dehydrating the 1-alcohol and/or cleavage of the dialkylether to obtain at least one alkene having 5 to 15 carbon atoms, wherein the at least one alkene having 5 to 15 carbon atoms is at least one olefin selected from the group consisting of: $\alpha$-, $\beta$- and/or $\gamma$-olefins, each independently having 5 to 15 carbon atoms, preferably the at least one alkene is a $\alpha$-olefin having a chain length of 5 to 15 carbon atoms;

wherein the dehydration and/or cleavage of step b) is performed with recirculation of dialkylether and/or 1-alcohol; and

wherein the dehydration and/or cleavage is performed at a temperature ranging from 255 °C to 290 °C;

c) reaction of the at least one alkene of step b) to obtain a product comprising or consisting of at least one alkanediol having 5 to 15 carbon atoms, preferably the product comprises or consist of at least one 1,2-alkanediol.

In a preferred variant, the present invention relates to a process for the preparation of alkanediols each independently having 5 to 15 carbon atoms, wherein in step c) of the process indicated above a product comprising or consisting of at least two alkanediols is obtained, wherein the at least two alkanediols are selected from the group consisting of: a 1,2-alkanediol, a 2,3-alkanediol and a 3,4-alkanediol, each independently having 5 to 15 carbon atoms, and wherein in the product, the ratio of the 1 ,2-alkanediol to the 2,3-alkanediol is in the range of 90 : 10 to 99.9 : 0.1 by weight and/or the ratio of the 1,2-alkanediol to the 3,4-alkanediol is in the range of 90 : 10 to 99.99 : 0.01 by weight. Thus, preferably the resulting product of step c) comprises or consists of a mixture of at least two isomeric (vicinal) alkanediols in specific ratios.

According to a preferred variant, the product of step c) of the process according to the present invention comprises or consists of a mixture of a 1,2-alkanediol and a 2,3-alkanediol, a mixture of a 1,2-alkanediol and a 3,4-alkanediol, a mixture of a 2,3-alkanediol and a 3,4-alkanediol, or a mixture of a 1,2-alkanediol, a 2,3-alkanediol and a 3,4-alkanediol, wherein the alkanediols in said mixtures have 5 to 15 carbon atoms each, and wherein said alkanediols preferably have the same chain length, i.e., they have the same number of carbon atoms but differ in their constitutional isomerism. Therefore, the mixture as specified herein comprises or consists of at least two alkanediols, wherein the number of the carbon atoms of said alkanediols is the same (homo combination).

This process allows for the preparation of alkanediols, and more specifically 1,2-alkanediols and/or specific alkanediol-mixtures as specified above, each independently having 5 to 15 carbon atoms using milder reaction conditions under reduced waste production in a more sustainable and economical manner, in particular during the preparation of the corresponding intermediate olefins according to step b) of the process. Based on the above more efficient and sustainable process, it is possible to prepare highly pure vicinal (x,x+1)-alkanediols, preferably 1,2-alkanediols, or mixtures of different (x,x+1)-alkanediols, while simultaneously allowing for a more gentle process with considerably reduced waste production and costs.

In a second aspect, the present invention relates to a composition comprising or consisting of at least two alkanediols selected from the group consisting of: a 1,2-alkanediol, a 2,3-alkanediol and a 3,4-alkanediol, each independently having 5 to 15 carbon atoms, wherein the ratio of the 1,2-alkanediol to the 2,3-alkanediol is in the range of 90 : 10 to 99.9 : 0.1 by weight and/or wherein the ratio of the 1,2-alkanediol to the 3,4-alkanediol is in the range of 90 : 10 to 99.99 : 0.01 by weight. Preferably, the product/composition comprises or consists of a 1,2-alkanediol and a 2,3-alkanediol, each independently having 5 to 15 carbon atoms, in the specified ratios.

Preferably said product/composition is obtained or obtainable according to the process of the present invention.

In a further aspect, the present invention relates to the use of the composition or product according to the invention for the preparation of consumer products such as cosmetics, personal care products, in particular skin cleaning products, shampoos, rinse-off conditioners, deodorants, antiperspirants, body lotions, homecare products, textile care products, household products, in particular liquid detergents, all-purpose cleaners, laundry and cleaning agents, fabric softeners, scent boosters, fragrance enhancers and pharmaceutical compositions as well as the consumer products as such comprising the composition according to the invention.

In another aspect the present invention relates to a process for the preparation of alkanediols, each independently having 5 to 15 carbon atoms, using at least one 1-alcohol having 5 to 15 carbon atoms in step a), and preferably at least two 1-alcohols having different chain lengths. In addition, the present invention relates to the obtained products (compositions) thereof, the use of said products for the preparation of consumer products and consumer products as such comprising the composition according to the invention.

Surprisingly it has been found that the overall process according to the present invention allows for the economically and ecologically efficient preparation of highly valuable alkanediols, especially a 1,2-alkanediol-based product or a mixture of a 1,2-alkanediol and a 2,3-alkandiol and/or a 3,4-alkandiol and in particular the preparation of the corresponding intermediate olefin compounds in a step b), with a reduced number of total process steps at moderate temperatures and with commercially available catalysts while simultaneously the amount of waste production is considerably reduced and allowing for an ideal balance of selectivity, yield and purity. Consequently, the process according to the invention allows for a more sustainable preparation of the desired compounds or compositions.

## Description of Figures

**Figures 1A to 1C:** Figures 1A to 1C show the experimental results of Experiment 1 in view of product composition (Figure 1A), conversion, selectivity, yield (Figure 1B) and purity (Figure 1C).

**Figures 2A to 2C:** Figures 2A to 2C show representative photographs of samples of 1,2-alkanediols (1), 2,3-alkanediols (2) and mixtures of 1,2-alkanediols with the respective 2,3-alkanediols in a ratio of 95 : 5 (3) of the corresponding heptanediols (Figure 2A), octanediols (Figure 2B) and nonanediols (Figure 2C), respectively.

**Figure 3:** Figure 3 is a diagram showing the ROS reducing capacity of the inventive alkanediols products and compositions.

## Detailed description of the invention

Primarily, the present invention relates to a process for the preparation of alkanediols having 5 to 15 carbon atoms as well as to mixtures of said alkanediols.

The alkanediols according to the present invention consist of a chain of 5 to 15 carbon atoms joined to each other by single covalent bonds with two OH-functional groups attached to two different carbon atoms in the chain. The term "alkanediol" within the context of the present invention also includes its constitutional isomers or position isomers. Constitutional isomers are compounds that have the same molecular formula and different connectivity. Position isomers, a particular form of constitutional isomerism, are structural isomers that can be viewed as differing only in the position of a functional group on a parent structure, which in this case is the position of the two alcohol functions.

Depending on the number of the carbon atoms of the carbon chain of the alkanediol, there are various position isomers of the alkanediol: (x,x+1)-constitutional isomers; (x,x+2)-constitutional isomers; (x,x+3)-constitutional isomers; etc., wherein x stands for the number of the carbon atom in the alkanediol chain to which the OH-groups of the alkanediol are chemically bonded.

In the (x,x+1)-constitutional isomers, the two functional alcohol groups (-OH) are vicinally attached to two different adjacent carbon atoms in the chain. In the (x,x+2)-constitutional isomers, the two OH-functional groups are attached to two different carbon atoms in the chain, wherein the two carbon atoms are separated by one carbon atom. In the (x,x+3)-constitutional isomers, the two OH-functional groups are attached to two different carbon atoms in the

chain, wherein the two carbon atoms are separated by two carbon atoms.

In a preferred variant, the alkanediols obtained or obtainable with the process according to the present invention are linear alkanediols having a carbon chain of 5 to 15 carbon atoms, and are preferably vicinal (x,x+1)-diols, selected from the group consisting of 1,2-diols, 2,3-diols and/or 3,4-diols or mixtures thereof.

Especially preferred, the main component of the product or the composition according to the second aspect, respectively, is a 1,2-alkanediol or a 1,2-alkanediol having as a minor component the respective 2,3- and/or 3,4-alkanediol analogues. Therefore, the present invention mainly focusses on the preparation of (x,x+1)-alkanediols and specifically on the preparation of high-purity 1,2-alkanediols or a mixture of a 1,2-alkanediol and a 2,3-alkandiol and/or a 3,4-alkandiol, preferably in specific ratios, and which each and independently are preferably linear in nature and have a chain length of 5 to 15 carbon atoms.

In a preferred variant, the product of step c) according to the first aspect of the present invention and the composition according to the second aspect of the present invention, respectively, is a product or composition comprising or consisting of a 1,2-alkanediol in its pure form, of a 1,2-alkanediol as the main component, or a mixture of a 1,2-alkanediol as the main component with minor portions of a 2,3-alkanediol and/or a 3,2-alkanediol. The product or composition according to the present invention can comprise minor portions of a corresponding 3,4-alkandiol, however, preferably no 3,4-alkandiol is present in the product of step c) according to the first aspect of the present invention and/or the composition according to the second aspect of the present invention. However, small amounts of the 3,4-analogues might be present in trace amounts as impurities.

In case of mixtures of two or more alkanediols, said alkanediols can be present either as a homo combination or alternatively as a hetero combination, in dependence of the corresponding chain lengths of the combined alkanediols. For example, if both, the first and the second alkanediol of a composition according to the present invention have the same number of carbon atoms, such an alkanediol combination is herein also referred to as "homo alkanediol mixture" or "homo combination". For example, the first linear alkanediol and the second linear alkanediol of the composition or product have a carbon chain of 7 carbon atoms, but the first linear alkanediol and the second linear alkanediol are different with regard to their constitutional isomerism or with regard to their stereoisomerism. If the first and the second alkanediol of the composition or product have a different number of carbon atoms, such an alkanediol combination is herein also referred to as "hetero alkanediol mixture" or "hetero combination". For example, the first linear alkanediol has a carbon chain of 7 carbon atoms and the second linear alkanediol has a carbon chain of 8 carbon atoms. However, beyond that, the first linear alkanediol and the second linear alkanediol can be different with regard to their constitutional isomerism or with regard to their stereoisomerism.

Preferably, the product of step c) according to the first aspect of the present invention and the composition according to the second aspect of the present invention, respectively, is preferably a product or composition wherein all components have the same chain lengths. In this case, said alkanediols are preferably differing in their constitutional isomerism, i.e. in view of the positions of the hydroxyl groups within the carbon chains. Such preferred combinations are for example products or compositions comprising or consisting of:

- a mixture of a 1,2-alkanediol and a 2,3-alkanediol having the same chain lengths (homo combination),
- a mixture of a 1,2-alkanediol and a 3,4-alkanediol having the same chain lengths (homo combination),
- a mixture of a 2,3-alkanediol and a 3,4-alkanediol having the same chain lengths (homo combination), or
- a mixture of a 1,2-alkanediol, a 2,3-alkanediol and a 3,4-alkanediol, wherein all three compounds have the same chain lengths (homo combination).

According to another preferred variant, the product of step c) according to the first aspect of the present invention or the composition according to the second aspect of the present invention is preferably a product or composition wherein all components have the same chain lengths. Such preferred combinations of alkanediols having chain lengths of 5 to 15 carbon atoms are for example products or compositions comprising or consisting of:

- a mixture of 1,2-pentanediol and 2,3-pentanediol, or
- a mixture of 1,2-hexanediol and 2,3-hexanediol, or
- a mixture of 1,2-heptanediol and 2,3-heptanediol, or
- a mixture of 1,2-octanediol and 2,3-octanediol, or
- a mixture of 1,2-nonanediol and 2,3-nonanediol, or
- a mixture of 1,2-decanediol and 2,3-decanediol, or
- a mixture of 1,2-undecanediol and 2,3-undecenaediol, or
- a mixture of 1,2-dodecanediol and 2,3-dodecanediol, or
- a mixture of 1,2-tridecanediol and 2,3-tridecanediol, or
- a mixture of 1,2-tetradecanediol and 2,3-tetradecanediol, or
- a mixture of 1,2-pentadecanediol and 2,3-pentadecanediol, or

- a mixture of 1,2-pentanediol and 3,4-pentanediol, or
- a mixture of 1,2-hexanediol and 3,4-hexanediol, or
- a mixture of 1,2-heptanediol and 3,4-heptanediol, or
- a mixture of 1,2-octanediol and 3,4-octanediol, or
- a mixture of 1,2-nonanediol and 3,4-nonanediol, or
- a mixture of 1,2-decanediol and 3,4-decanediol, or
- a mixture of 1,2-undecanediol and 3,4-undecenaediol, or
- a mixture of 1,2-dodecanediol and 3,4-dodecanediol, or
- a mixture of 1,2-tridecanediol and 3,4-tridecanediol, or
- a mixture of 1,2-tetradecanediol and 3,4-tetradecanediol, or
- a mixture of 1,2-pentadecanediol and 3,4-pentadecanediol, or
- a mixture of 2,3-pentanediol and 3,4-pentanediol, or
- a mixture of 2,3-hexanediol and 3,4-hexanediol, or
- a mixture of 2,3-heptanediol and 3,4-heptanediol, or
- a mixture of 2,3-octanediol and 3,4-octanediol, or
- a mixture of 2,3-nonanediol and 3,4-nonanediol, or
- a mixture of 2,3-decanediol and 3,4-decanediol, or
- a mixture of 2,3-undecanediol and 3,4-undecenaediol, or
- a mixture of 2,3-dodecanediol and 3,4-dodecanediol, or
- a mixture of 2,3-tridecanediol and 3,4-tridecanediol, or
- a mixture of 2,3-tetradecanediol and 3,4-tetradecanediol, or
- a mixture of 2,3-pentadecanediol and 3,4-pentadecanediol, or
- a mixture of 1,2-pentanediol, 2,3-pentanediol and 3,4-pentanediol, or
- a mixture of 1,2-hexanediol, 2,3-hexanediol and 3,4-hexanediol, or
- a mixture of 1,2-heptanediol, 2,3-heptanediol and 3,4-heptanediol, or
- a mixture of 1,2-octanediol, 2,3-octanediol and 3,4-octanediol, or
- a mixture of 1,2-nonanediol, 2,3-nonanediol and 3,4-nonanediol, or
- a mixture of 1,2-decanediol, 2,3-decanediol and 3,4-decanediol, or
- a mixture of 1,2-undecanediol, 2,3-undecanediol and 3,4-undecenaediol, or
- a mixture of 1,2-dodecanediol, 2,3-dodecanediol and 3,4-dodecanediol, or
- a mixture of 1,2-tridecanediol, 2,3-tridecanediol and 3,4-tridecanediol, or
- a mixture of 1,2-tetradecanediol, 2,3-tetradecanediol and 3,4-tetradecanediol, or
- a mixture of 1,2-pentadecanediol, 2,3-pentadecanediol and 3,4-pentadecanediol.

In the following the different isomeric forms are exemplarily shown for a compound having 7 carbon atoms:

1,2-Heptandediol belongs to the category of alkanediols and is a straight chain vicinal alkanediol with the general formula:

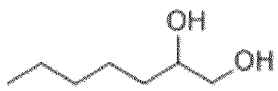

It has a carbon chain with 7 carbon atoms and the two functional OH-groups of the alkanediol are in alpha, beta-position and chemically bonded to the C1 and C2 carbon atoms in the alkanediol chain.

2,3-Heptanediol also belongs to the category of alkanediols and is a straight chain vicinal alkanediol with the general formula:

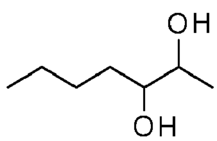

It has a carbon chain with 7 carbon atoms and the two functional OH-groups of the alkanediol are in beta, gamma-position and chemically bonded to the C2 and C3 carbon atoms in the alkanediol chain.

More specifically, in a first aspect, the present invention relates to a process for the preparation of alkanediols having 5 to 15 carbon atoms, and preferably comprising or consisting of a 1,2-alkanediol or a mixture of a 1,2-alkanediol and a 2,3-alkanediol and/or a 3,4-alkanediol, comprising the following steps:

a) providing a 1-alcohol having 5 to 15 carbon atoms and/or a dialkylether having 10 to 30 carbon atoms and a catalyst;
b) dehydrating the 1-alcohol and/or cleavage of the dialkylether to obtain at least one alkene having 5 to 15 carbon atoms, wherein the at least one alkene having 5 to 15 carbon atoms is at least one olefin selected from the group consisting of: α-, β- and/or γ-olefins, each independently having 5 to 15 carbon atoms, and preferably an α-olefin having a chain length of 5 to 15 carbon atoms;

wherein the dehydration and/or cleavage of step b) is performed with recirculation of dialkylether and/or 1-alcohol; and
wherein the dehydration and/or cleavage of step b) is performed at a temperature ranging from 255 °C to 290 °C;

c) reaction of the at least one alkene of step b), preferably a α-olefin, to obtain a product comprising or consisting of at least one alkanediol having 5 to 15 carbon atoms, preferably a 1,2-alkanediol.

In a preferred variant the present invention relates to a process for the preparation of alkanediols having 5 to 15 carbon atoms, wherein in step c) of the process indicated above a product is obtained comprising or consisting of at least two alkanediols selected from the group consisting of: a 1,2-alkanediol, a 2,3-alkanediol and a 3,4-alkanediol, each independently having 5 to 15 carbon atoms, wherein in the product of step c), the ratio of the 1,2-alkanediol to the 2,3-alkanediol is in the range of 90 : 10 to 99.9 : 0.1 by weight and/or the ratio of the 1 ,2-alkanediol to the 3,4-alkanediol is in the range of 90 : 10 to 99.99 : 0.01 by weight.

The as-obtained or obtainable products or compositions comprising or consisting of a 1,2-alkanediol or a 1,2-alkanediol as main component in a mixture of alkanediols, and in particular the 1,2-heptanediol, 1,2-octanediol or 1,2-nonanediol-based product with optionally or preferably the corresponding 2,3-heptanediol, 2,3-octanediol or 2,3-nonanediol and/or the corresponding 3,4-heptanediol, 3,4-octanediol or 3,4-nonanediol, is neutral in odour and obtained in liquid form allowing for the facilitated and advantageous incorporation of said substances and mixtures (compositions) obtained or obtainable by said process into a variety of product formulations without negatively affecting the final product properties such as the texture or odour. Correspondingly, the product or composition preferably obtained in step c) of the process according to the present invention is a 1,2-alkanediol having 6 to 9 carbon atoms or a product comprising or consisting of a composition/mixture of at least two alkanediols selected from the group consisting of: a 1,2-alkanediol, a 2,3-alkanediol and a 3,4-alkanediol, each independently having 7 to 9 carbon atoms.

Likewise, the composition according to the second aspect of the present invention is preferably a composition comprising or consisting of vicinal alkanediols, each independently having 7 to 9 carbon atoms, and in particular, preferably, is 1,2-heptanediol, 1,2-octanediol or 1,2-nonanediol-based, optionally or preferably with the corresponding 2,3-heptanediol, 2,3-octanediol or 2,3-nonanediol and/or the corresponding 3,4-heptanediol, 3,4-octanediol or 3,4-nonanediol. Even more preferred the composition comprises or consists of 1,2-heptanediol, 1,2-octanediol or 1,2-nonanediol, respectively, with the corresponding 2,3-heptanediol, 2,3-octanediol or 2,3-nonanediol without further 3,4-components or optionally wherein the corresponding 3,4-components are present in amounts of less than 0.5 % by weight and preferably less than 0.25 % by weight.

In a preferred variant thereof, the 1,2-alkanediol and the 2,3-alkanediol (and optionally the 3,4-alkanediol) have the same chain lengths, i.e., they bear the same number of carbon atoms. Preferably, they compound have chain lengths of 7 or 8 carbon atoms each.

In addition, it was observed, that the corresponding 2,3-alkanediols (and/or 3,4-alkanediols) as such are neutral in odour and obtainable in liquid form, likewise allowing for the advantageous incorporation into various product formulations. Moreover, it was found that the combination of 1,2-alkanediols with 2,3-alkanediols results in advantageous effects in a synergistical manner as shown in the experimental section below.

In a preferred variant the product/composition thus comprises or consists of a combination of a 1,2-alkanediol as first linear alkanediol and the corresponding 2,3-alkanediol as second linear alkanediol, such as in:

- a mixture of 1,2-pentanediol and 2,3-pentanediol, or
- a mixture of 1,2-hexanediol and 2,3-hexanediol, or
- a mixture of 1,2-heptanediol and 2,3-heptanediol, or
- a mixture of 1,2-octanediol and 2,3-octanediol, or
- a mixture of 1,2-nonanediol and 2,3-nonanediol, or
- a mixture of 1,2-decanediol and 2,3-decanediol, or
- a mixture of 1,2-undecanediol and 2,3-undecenaediol, or
- a mixture of 1,2-dodecanediol and 2,3-dodecanediol, or
- a mixture of 1,2-tridecanediol and 2,3-tridecanediol,
- a mixture of 1,2-tetradecanediol and 2,3-tetradecanediol, or
- a mixture of 1,2-pentadecanediol and 2,3-pentadecanediol,

the first linear alkanediol and the second linear alkanediol being present in a ratio ranging from 90 : 10 to 99.9 : 0.1.

Optionally, the corresponding 3,4-compound is present as third linear alkanediol, preferably within the ratios specified herein.

Based on such a combination of a 1,2-alkanediol as first linear alkanediol and the corresponding 2,3-alkanediol as second linear alkanediol, significant improvements in the chemical and physical properties of the mixtures could be observed compared to the pure single substances. In particular, advantageous effects could be observed for the combination of a 1,2-alkanediol as the main portion of the composition and minor portions of the corresponding 2,3-alkanediol, i.e. the 2,3-alkanediol having the same chain length as the 1,2-alkanediol (homo combination). Thus, compositions comprising or consisting of homo combinations of a 1,2-alkanediol as the main portion of the composition and the corresponding 2,3-alkanediol in a minor portion are particularly preferred within the context of the first and second aspects of the present invention. Even more preferred, the alkanediols of said composition are having chain lengths of 5 to 9, preferably of 6 to 9 and even more preferred of 7 or 8 carbon atoms.

According to further preferred variant, in such compositions the corresponding 3,4-alkanediol is present in amounts of less than 0.5 % by weight and preferably less than 0.25 % by weight.

Therefore, the product of step c) according to the first aspect of the present invention and/or the composition according to the second aspect of the present invention is preferably a product or composition comprising or consisting of:

- 1,2-pentanediol and 2,3-pentanediol, or
- 1,2-hexanediol and 2,3-hexanediol, or
- 1,2-heptanediol and 2,3-heptanediol, or
- 1,2-octanediol and 2,3-octanediol, or
- 1,2-nonanediol and 2,3-nonanediol,

wherein the combinations of 1,2-heptanediol and 2,3-heptanediol, or 1,2-octanediol and 2,3-octanediol are particularly preferred, and wherein optionally only minor amounts of the corresponding 3,4-compounds are present.

Figures 2A to 2C show representative photographs of samples of 1,2-alkanediols (1), 2,3-alkanediols (2) and mixtures of 1,2-alkanediols with the respective 2,3-alkanediols in a ratio of 95 : 5 (3) of the corresponding heptanediols (Figure 2A), octanediols (Figure 2B) and nonanediols (Figure 2C), respectively. All mixtures are liquid, while pure 1,2-octanediol and 1,2-nonanediol are obtainable in solid form. Minor amounts of the corresponding 2,3-isomers (about 5 % by weight are required) allow for the preparation of colourless and liquid mixtures with improved properties which can efficiently be incorporated into a wide variety of possible applications. Additionally, with the admixture of the corresponding liquid 2,3-alkanediol, even in small amounts, to a solid 1,2-alkanediol, the solid 1,2-alkanedioles, such as 1,2-octanediol, 1,2-nonanediol etc., can be solved, resulting in a liquid alkanediol mixture. The 2,3-alkanediol serves as solvent for the solid 1,2-alkanediols. Such liquid mixtures have the benefit that firstly, the availability of the solid 1,2-alkanediol in the mixture is improved and secondly, the incorporation of the solid 1,2-alkanediol in semi-finished products or final products is facilitated. This effect is particularly favourably for emulsions, in which lipophilic 1,2-alkanediols having a carbon chain of 8 or more carbon atoms when used alone, tend to migrate into the oil phase or tend to precipitate or recrystallize.

Additionally, it was found that 1,2-octanediol tends to precipitate or recrystallize in sunflower oil. Unlike 1,2-octanediol, 2,3-octanediol has surprisingly a good solubility in sunflower oil. Interestingly, mixtures of 1,2-octanediol and 2,3-octanediol comprising minor portions of the 2,3-alkanediol (preferably in the ratios specified above) have a good solubility and lead to clear solutions. Thereby, less than 5 % by weight of the 2,3-alkanediol relative to 95 % by weight of the 1,2-alkanediol were sufficient in order to achieve significantly improved solubilities of the 1,2-alkanediol.

It was further surprisingly found that 1,2-heptanediol and 2,3-heptanediol in a ratio of 95 : 5 show a synergistic antimicrobial efficacy against *Candida albicans.* Additionally, specific combinations of 1,2-alkanediols with 2,3-alkanediols show synergistic effects in terms of sebum control, malodour management, and modulation of fragrance notes, modification of fragrance notes and suppression of fragrance notes.

In particular, the advantageous synergistic effects were observed for homo combinations of 1,2-alkanediols with 2,3-alkanediols (preferably in absence of the corresponding 3,4-analogue), but also hetero combinations of 1,2-alkanediol with 2,3-alkanediols showed advantages for the preparation of consumer product formulations.

In a preferred variant in step c) of the inventive process a product is obtained which comprises or consists of at least two alkanediols selected from the group consisting of: a 1,2-alkanediol, a 2,3-alkanediol and a 3,4-alkanediol, each independently having 5 to 15 carbon atoms, i.e., the product preferably comprises or consists of a combination of a 1,2-alkanediol with a 2,3-alkanediol, a combination of a 1,2-alkanediol with a 3,4-alkanediol, a combination of a 2,3-alkanediol and a 3,4-alkanediol, or a combination of a 1,2-alkanediol, a 2,3-alkanediol and a 3,4-alkanediol. Thereby, the alkanediols independently have chain lengths of 5 to 15 carbon atoms, wherein the chain lengths of the single alkanediols are either different or the same, however, preferably, the chain lengths of the alkanediols are the same in the composition/product. Moreover, in the product, preferably the ratio of the 1,2-alkanediol to the 2,3-alkanediol is in the range of 90 : 10 to 99.9 : 0.1 and/or the ratio of the 1,2-alkanediol to the 3,4-alkanediol is in the range of 90 : 10 to

99.99 : 0.01 by weight.

In a further preferred variant of the present invention the product preferably comprises or consists of a combination of a 1,2-alkanediol with a 2,3-alkanediol in the ratios specified above.

The formation of side products such as esters and dioxanes which might cause disadvantageous smells was not observed when preparing alkanediols according to the process specified herein.

Alternatively, based on the present process, it is possible to prepare highly pure 1,2-alkanediols having chain lengths of 5 to 15 carbon atoms, such as 1,2-pentanediol, 1,2-hexanediol, 1,2-heptanediol, 1,2-octanediol, 1,2-nonanediol, 1,2-decanediol, 1,2-undecanediol, 1,2-dodecanediol, 1,2-tridecanediol, 1,2-tetradecanol, or 1,2-pentadecanol.

With the low temperature process according to the invention, it was further possible to achieve high-quality alkanediols without the requirement of additional complicated purification processes.

The term "comprising" means that the named components are essential, but other components may be added and are still embraced by the present invention while the term "consisting of" as used according to the present invention means that the total amount of components adds up to 100 % by weight, based on the total weight of the corresponding product or composition, and signifies that the subject matter is closed-ended and can only include the limitations that are expressly recited. Whenever reference is made to "comprising" it is intended to cover both meanings as alternatives, that is the meaning can be either "comprising" or "consisting of", unless the context dictates otherwise.

The terms "at least one" and "at least two" mean that the corresponding product or composition according to the present invention can comprise either one or two or a mixture of two or more of the corresponding components, respectively. The term "optionally" means that the subsequently described compound may but need not to be present in the composition, and that the description includes variants, wherein the compound is included or variants, wherein the compound is absent, respectively.

The product obtainable in step c) of the process according to the invention comprises or consists of at least one alkanediol having 5 to 15 carbon atoms which is preferably a 1,2-alkanediol, or the product obtainable in step c) is a composition that comprises or consist of a mixture of at least two alkanediols selected from the group consisting of: a 1,2-alkanediol, a 2,3-alkanediol and a 3,4-alkanediol, each independently having 5 to 15 carbon atoms. If the desired product is a 1,2-alkanediol, relatively low portions of 2,3- and/or 3,4-alkanediols in the final product are preferred, corresponding to high-purity 1,2-alkanediols having 5 to 15 carbon atoms or alternatively, advantageous mixtures/compositions of 1,2-alkanediols with 2,3-alkanediols and/or 3,4-alkanediols within the ratios specified below can be prepared based on the inventive process. Consequently, in step b) of the process according to the invention preferably high amounts of the corresponding $\alpha$-olefins are formed while the amounts of the corresponding $\beta$- and/or $\gamma$-olefins in the intermediate product of step b) are comparatively low.

According to a preferred variant of the present invention, even more preferred, in such cases the only olefin formed in step b) of the process according to the invention is a $\alpha$-olefin. Thus, preferably high-purity $\alpha$-olefins are formed in step b) of the process according to the invention. However, depending on the reaction conditions it is also possible to adjust the process accordingly to predominantly, or exclusively, obtain $\beta$-alkenes in step b) for the preparation of high-purity 2,3-alkanediols in step c) or alternatively $\gamma$-alkenes for the preparation of high-purity 3,4-alkanediols in step c), respectively.

For mixtures of at least two alkanediols and thus compositions as specified above, correspondingly the ratios of the components of the mixture are reflected in the corresponding olefin-compounds of step b) of the process.

Thereby, in the case of mixtures, the ratio of the 1,2-alkanediol to the 2,3-alkanediol of the composition obtained by the inventive process, and more specifically in step c) of said process, is in the range of 90 : 10 to 99.9 : 0.1 and/or the ratio of the 1,2-alkanediol to the 3,4-alkanediol is in the range of 90 : 10 to 99.99 : 0.01. Preferably, however, the ratio of the 1,2-alkanediol to the 2,3-alkanediol is in the range of 95 : 5 to 99.9 : 0.1 and preferably from 97.5 : 2.5 to 99.9 : 0.1 and/or the ratio of the 1,2-alkanediol to the 3,4-alkanediol is in the range of 95 : 5 to 99.99 : 0.01 and preferably from 97.5 : 2.5 to 99.99 : 0.01. Thereby, in a particularly preferred variant of the present invention the ratio of the 1,2-alkanediol to the 2,3-alkanediol is in the range of 90 : 10 to 99.9 : 0.1, preferably in the range of 95 : 5 to 99.9 : 0.1 and even more preferred from 97.5 : 2.5 to 99.9 : 0.1, whereby very low amounts of the corresponding 3,4-alkanediol are present in the composition obtained by the inventive process, and more specifically in step c) of said process, and wherein the isomeric alkanediols have the same chain lengths (homo combination).

Thus, preferably the two isomeric alkanediols are present in the mixture according to the present invention in a ratio in a range of 90 : 10 to 99.9 : 0.1, even more preferred in a ratio in a range of 95 : 5 to 99.9 : 0.1 or in the range of 97.5 : 2.5 to 99.9 : 0.1. In said homo alkanediol mixtures the first linear alkanediol (1,2-alkanediol) and the second linear alkanediol (2,3-alkanediol) have the same number of carbon atoms.

In an advantageous variant according to the second aspect of the present invention, the mixture comprises as first linear alkanediol an 1,2-alkanediol, such as 1,2-pentanediol, 1,2-hexanediol, 1,2-heptanediol, 1,2-octanediol, 1,2-nonanediol, 1,2-decanediol, 1,2-undecanediol, 1,2-dodecanediol, 1,2-tridecanediol, 1,2-tetradecanediol or 1,2-pentadecanediol, and as second linear alkanediol the corresponding isomeric 2,3-alkanediols, such as 2,3-alkanediol, 2,3-hexanediol, 2,3-heptanediol, 2,3-octanediol, 2,3-nonanediol, 2,3-decanediol, 2,3-undecanediol, 2,3-dodecanediol, 2,3-tridecanediol, 2,3-tetradecanediol or 2,3-pentadecanediol in a ratio in a range of 90 : 10 to 99.9 : 0.1, preferably in a ratio in a range of 95 : 5 to 99.9 : 0.1, and more preferred in a ratio in a range of 97.5 : 2.5 to 99.9 : 0.1.

Thus, in a further preferred variant, the product of the process according to the invention comprises or consists of a mixture of a 1,2-alkanediol as the first linear alkanediol and a 2,3-alkanediol as the second linear alkanediol and/or a 3,4-alkanediol as the third linear alkanediol, with preferably only minor portions of 2,3- and/or 3,4-alkanediols in the ratios specified above or in another preferred variant the final product of step c) is a high-purity 1,2-alkanediol, a high-purity 2,3-alkanediol or a high-purity 3,4-alkanediol, but preferably a high-purity 1,2-alkanediol having a chain length of 5 to 15 carbon atoms.

In a preferred variant of the present invention, the ratio of the 1,2-alkanediol to the 2,3-alkanediol and/or the 3,4-alkanediol is high, corresponding to a high chemical purity of the product, i.e. the 1,2-alkanediol. Preferably, the purity of the 1,2-alkanediol obtainable by the inventive process is 95 % or higher, preferably 96 % or higher, even more preferred 97 % or higher, especially preferred 98 % or higher and most preferred 99 % or higher. By additional distillation of the products easily product purities of up to 99.9 % can be achieved.

Based on the process according to the invention, it is possible to achieve high-purity 1,2-alkanediols or composition comprising or consisting of mixtures of 1,2-alkanediols with low amounts of the corresponding 2,3-alkanediols and/or 3,4-alkanediols as side products, wherein the 2,3-alkanediols form the main portion of the side products, i.e., extremely low portions of 3,4-alkanediols are present in the final product/composition. Preferably however, no 3,4-alkanediol is present in the product/composition. For special applications, it can also be favourable to have a certain amount of 2,3- and/or 3,4-components in the composition. Such mixtures can have good skin spreading properties, possibly due to the more diverse spectrum of components which have a broader interaction with the other components in topical formulations. Within the context of the present invention, it was for example found, that minor portions of 2,3-alkanediols highly improve the properties of 1,2-alkanediols in a synergistic manner.

For example, by using a mixture comprising 1,2-heptanediol and 2,3-heptanediol or a mixture of 1,2-octanediol and 2,3-octanediol in a ratio of 95 : 5 the spreading behaviour could significantly be increased and/or the fatty/greasy skin feel of oily ingredients could significantly be reduced, in particular in view of liquid lipophilic components which have a poor spreading by nature.

All of the afore specified homo alkanediol mixtures including a 1,2-alkanediol and minor portions of the corresponding 2,3-alkanediol display a significant solubility improvement for lyophilic active substances and are clearly superior to the individually corresponding 1,2-alkanediols or 2,3-alkanediols, having the same concentration, but which show a poorer solubility. All solutions of said 1,2-alkanediol and 2,3-alkanediol mixtures with a lipophilic active component are clear and stable and the lipophilic active substance does not precipitate out even during storage.

In addition, it was surprisingly found that mixtures, such as mixtures of 1,2-octanediol and minor portions of 2,3-octanediol or mixtures of 1,2-heptanediol and minor portions of 2,3-heptanediol, surprisingly have a good solubility in oils and lead to clear solutions, while for example, 1,2-octanediol tends to precipitate or recrystallize in sunflower oil, indicating a beneficial and synergistical effect of the additional 2,3-component. This advantages effect could be observed even if only minor amounts of the 2,3-component were added, so that samples of a mixture of 1,2-octanediol and 2,3-octanediol in a ratio of 95 : 5 or 1,2-heptanediol and 2,3-heptanediol in a ratio of 95 : 5 already showed considerably improved solubilities in the oil phase. Furthermore, for example, the blend of caprylic capric triglycerides (CCT) with 1,2-hexandiol and 2,3-hexanediol in a ratio 95 : 5 showed a less fatty/greasy skin feel and in addition less remaining residue on skin (indicates better absorption) versus 1,2-hexanediol and 2,3-hexanediol alone. Also the blend of cetearyl nonanoate with 1,2-heptanediol and 2,3-heptanediol in a ratio 95 : 5 showed significant better spreading properties versus 1,2-heptanediol and 2,3-heptanediol alone. Moreover, the blend of octocrylene with 1,2-heptanediol and 2,3-heptanediol in a ratio 95 : 5 or a blend of octocrylene with 1,2-octanediol and 2,3-octanediol in a ratio 95 : 5, respectively, showed better spreading properties, less fatty/greasy skin feel and better absorption (i.e. less remaining residue on skin) versus the blend of octocrylene with 1,2- and 2,3-heptanediol each alone or the blend of octocrylene with 1,2- and 2,3-octanediol each alone, respectively.

In addition, said homo combinations comprising or consisting of 1,2-alkanediols and 2,3-alkanediols can be stably incorporated into a broad variety of formulations.

The purity and composition of the final alkanediol compound is essentially reflected in the purity and composition of the intermediary prepared alkene compounds of step b). Accordingly, in step b) of the inventive process, preferably high-purity 1-alkenes ($\alpha$-olefins) are obtained with only minor amounts of 2- and/or 3-alkenes ($\beta$- and/or $\gamma$-olefins) in order to prepare high-purity 1,2-alkanediols. Preferably, only 1-alkenes are formed in step b) of the process according to the invention. Alternatively, the 1-alkenes are obtained with minor amounts of 2- and/or 3-alkenes according to the ratios specified herein for the preparation of synergistic compositions of 1,2-alkanediols and 2,3-alkanediols (and/or 3,4-alkanediols) in homo combinations.

Accordingly, in a preferred variant wherein the product comprises at least two alkanediols the proportion of the 1-alkene to the 2-alkene at the end of step b) is preferably in the range of 90 : 10 to 99.9 : 0.1 and/or the ratio of the 1-alkene to the 3-alkene is in the range of 90 : 10 to 99.99 : 0.01. Preferably, the ratio of the 1-alkene to the 2-alkene is

in the range of 95 : 5 to 99.9 : 0.1 and preferably from 97.5 : 2.5 to 99.9 : 0.1 and/or the ratio of the 1-alkene to the 3-alkene is in the range of 95 : 5 to 99.99 : 0.01 and preferably from 97.5 : 2.5 to 99.99 : 0.01.

Furthermore, in a preferred variant the purity of the 1-alkene is preferably 95 % or higher and preferably 96 % or higher, even more preferred 97 % or higher, especially preferred 98 % or higher and most preferred 99 % or higher. Additionally, the purity of the 1-alkene can be further increased by standard distillation techniques if required up to purities of about 99.9 % allowing for the preparation of high-purity alkanediols, and preferably 1,2-alkanediols, in step c) according to the inventive process.

The process described herein is characterized in particular by its high efficiency, as the process in question is preferably a so-called "self-regulating" process in which in a preferred variant with recirculation, a steady state can be achieved. This process equilibrium can be positively influenced by optimization of the recirculation rate and the selected reaction temperature. Additional variations of the residence time above the catalyst can, for example, also influence the product formation. In particular, it has been observed that the recycling rate as well as the low temperatures as described herein have a positive effect on the formation of 1,2-alkanediols as the main product. Recirculation rates of the 1-alcohol and/or dialkylether of approximately 40 % (+/- 10 %) and temperatures below 300 °C enable the efficient and economically as well as ecologically improved formation of high-purity 1,2-alkanediols or mixtures of 1,2-alkanediols with lower levels of 2,3- and/or 3,4-alkanediols as specified herein.

Moreover, the alkanediols according to the invention having chain lengths of 5 to 15 carbon atoms are preferably selected from the group consisting of pentanediols, hexanediols heptanediols, octanediols, nonanediols, decanediols, undecanediols, dodecanediols, tridecanediols, tetradecanediols and pentadecanediols in the corresponding isomeric forms as defined above.

In a preferred variant, however, the corresponding alkanediol is selected from the group consisting of pentanediol (C5), hexanediol (C6), heptanediol (C7), octanediol (C8) and nonanediol (C9). Therefore, in a preferred variant a process according to the first aspect for the preparation of alkanediols having 5 to 9 carbon atoms is disclosed. Preferably the alkanediols have chain lengths of 6 to 9 carbon atoms.

Consequently, step c) of the process according to the first aspect relates to the reaction of at least one alkene to obtain a product comprising or consisting of at least one alkanediol having 6 to 9 carbon atoms or a mixture of at least two alkanediols selected from the group consisting of: 1,2-alkanediols, 2,3-alkanediols and 3,4-alkanediols, each independently having 6 to 9 carbon atoms in the corresponding ratios specified above.

However, in a still more preferred variant, the process as described herein relates to the preparation of alkanediols having 7 or 8 carbon atoms, and preferably 7 carbon atoms. Therefore, in a further preferred variant a product comprising or consisting of at least one alkanediol having 7 or 8 carbon atoms or a mixture of at least two alkanediols selected from the group consisting of: 1,2-alkanediols, 2,3-alkanediols and 3,4-alkanediols, each independently having 7 to 8 carbon atoms, and preferably 7 carbon atoms, is obtainable, i.e. a product comprising or consisting of at least one alkanediol being 1,2-heptanediol or 1,2-octanediol or a mixture of at least two alkanediols selected from the group consisting of: 1,2-heptanediol, 2,3-heptanediol and 3,4-heptanediol and/or 1,2-octanediol, 2,3-octanediol and 3,4-octanediol, respectively, in the ratios specified above.

Therefore, in a first step a) a 1-alcohol having 5 to 15 carbon atoms, and preferably 5 to 9, more preferred 6 to 9 and most preferred 8 to 9 carbon atoms, as well as a suitable catalyst are provided. Additionally, or optionally, a dialkylether having 10 to 30 carbon atoms is provided. Preferably, only a 1-alcohol having 5 to 15 carbon atoms as well as a suitable catalyst is provided as starting material.

The 1-alcohol is thereby influencing the chain length of the corresponding final alkanediols and is preferably linear in nature. The 1-alcohol and/or the dialkylether are thus selected in dependence of the aimed product composition.

Preferred 1-alcohols used for the process according to the invention are therefore preferably linear, saturated 1-alkanols having 5 to 15 carbon atoms, preferably 6 to 9 carbon atoms and even more preferred 7 or 8 carbon atoms, such as 1-pentanol, 1-hexanol, 1-heptanol, 1-octanol, 1-nonanol, 1-decanol, 1-undecanol, 1-dodecanol, 1-tridecanol, 1-tetradecanol and 1-pentadecanol, wherein 1-hexanol, 1-heptanol, 1-octanol and 1-nonanol are preferred. Alkanols are a group of substances derived from the alkanes (saturated hydrocarbons).

Thereby, the alcohol provided in step a) is either freshly added and/or recirculated unreacted or formed alcohol from step b).

Preferably, said 1-alkanoles used within the context are preferably obtained from natural sources and thus have a natural origin. For example, 1-pentanol can be isolated from various plants, such as Capsicum frutescens, raspberries, tomatoes, blueberries, cabbage, spearmint, watermelon, grapefruit, and rosemary, while 1-hexanol can be found in strawberries, bananas, tomatoes, garlic, apples, parsley, soybeans and the like. 1-Heptanol is a substance which is amongst others naturally occurring in apples, celery, rooibos, black walnut, common wheat, lemon peel, numerous nuts and potato plants. Naturally occurring 1-octanol is found among others in strawberries, wild strawberries, anise scented nettle, Boswellia sacra, tea, St. John's wort, and ginger. 1 -Nonanol is naturally common, for example, in orange oil, grapefruit, strawberries, clary sage and ginger. Naturally, 1-decanol occurs in various plants, such as Houttuynia cordata, the almond tree, apple trees, horse chestnut, cilantro, spearmint, spice vanilla, and corn. The alkanols

of step a) can also be derived from natural oils and fatty acid esters (such as coconut, castor etc.) by processing and chemical transformation like cracking, pyrolysis, transesterification, or retro-Prins-type reactions.

Preferred dialkylethers used for the process according to the invention are organic compounds that have an ether group as their functional group, i.e., an oxygen atom substituted with two alkyl radicals (R1-O-R2). The alkyl radicals R1 and R2 can be the same or different from each other resulting in either symmetrical dialkylethers or asymmetrical ones. Consequently, each alkyl radical has independently 5 to 15 carbon atoms corresponding to 10 to 30 carbon atoms in total. The use of symmetrical dialkylethers results in the formation of olefins all having the same chain length while dialkylethers having two different alkyl radicals cause the formation of olefins having different chain lengths corresponding to the chain lengths of the respective alkyl radicals of the dialkylether. Consequently, it is possible to obtain mixtures of olefins differing in chain length and thus hetero combinations of alkanediols. A comparable effect can be achieved by providing a mixture of different 1-alcohols each differing in chain length. Thereby, the dialkylether provided in step a) is either freshly added dialkylether and/or recirculated dialkylether formed in step b) as intermediate itself in the formation of the intermediate olefin compounds for preparing the corresponding final alkanediol product in step c) or unreacted dialkylether.

Suitable catalysts used for the process according to the present invention are preferably commercially available and are preferably alumina based. However, generally any suitable catalyst known in the art can be used in the dehydration step b) of the process according to the invention.

In addition, the catalyst used for the process according to the present invention is preferably unmodified, i.e., neither doped with metals such as zirconium, zinc or other metal atoms or other additives or acids and bases nor processed or treated in any other physical or chemical way prior to utilization.

Usually, the selectivity of a reaction can be increased by using doped or modified catalysts. However, due to a decrease in reactivity generally higher reaction temperatures are required. These high temperatures have a negative effect on the durability of the reaction equipment increasing wear of the equipment and resulting in additional costs for device inspections. Moreover, such high temperatures require expensive insulations rendering the equipment more expensive as such and temperatures above 300 °C are hard to achieve using oil-based heating so that very expensive liquid salt melts would have to be used for heating. In addition, such modifications are mostly rather complex and costly and cause additional chemical waste. Consequently, high temperatures are on the one hand economically inefficient and ecologically non-sustainable and on the other hand, if no specific catalyst is used, result in reduced product selectivity due to the formation of side products. However, the use of specifically modified catalysts requires additional reaction steps and often very high calcination temperatures so that these catalysts do not seem an appropriate solution.

However, it has now been found that commercially available non-modified catalysts can be used to achieve high-purity alkanediols or alkanediol mixtures/compositions as specified above at moderate reaction conditions based on the process according to the invention which itself is based on the recirculation of intermediary formed products and non-reacted educts. Therefore, the present invention preferably employs a non-modified catalyst.

Preferably, the non-modified catalyst used in the process according to the invention, is a catalyst comprising or consisting of alumina ($Al_2O_3$) such as high purity gamma ($\gamma$-$Al_2O_3$), transitional ($\delta$-$Al_2O_3$ and $\theta$-$Al_2O_3$) and alpha alumina ($\alpha$-$Al_2O_3$) or $\eta$-$Al_2O_3$ in the form of powders, granules, spheres, high density pellets and the like. Preferably, the catalyst has a pore volume ranging from 0.2 to 1.5 ml/g and even more preferred from 0.35 to 1.0 ml/g. A catalyst having a surface area of 120 to 250 $m^2$/g and even more preferred of 130 to 225 $m^2$/g is also suitable.

Preferably, unmodified $\gamma$-alumina is used as the catalyst, which is commercially available and does not require additional modifications prior to use.

Therefore, according to a further preferred variant, a process for the preparation of alkanediols is disclosed, wherein in step a) the used catalyst is an unmodified $\gamma$-alumina-based catalyst.

It is therefore a further object of the present invention, to provide a process for preparing alkanediols, and preferably 1,2-alkanediols or mixtures thereof, and in particular the corresponding intermediary formed olefins by dehydration of alcohols and/or cleavage of dialkylether, which is more sustainable and has a good balance between yield, selectivity and purity and simultaneously a reduced waste production at moderate temperatures of less than 300 °C allowing for the ecologically-friendly and economically efficient preparation of the desired alkanediols, especially the 1,2-alkane products with high purities or alkanediol mixtures as specified herein. Thereby, preferably no additional modification of the catalyst is required.

Based on the gentle process and the recycling according to the invention, it is possible to reduce the amount of waste produced to a minimum, such as dialkylethers or non-reacted educts which would otherwise be lost to the production. Furthermore, other types of waste such as formed in exotic side-reactions such as carbon backbone rearrangements are highly suppressed in the present inventive process.

Presently, intermediary formed dialkylether and/or non-reacted dialkylether in the preparation of the corresponding alkenes are fully recycled and only extremely low amounts of 2-alkenes (and consequently the corresponding 2,3-alkanediols) are formed. However, the present process allows for modest or even low 2-alkene formation and especially even lesser amounts of 3-alkenes and their formation. Thus, only extremely low amounts of 2-alkenes, and/or

of 3-alkenes (and thus the corresponding 2,3- and/or 3,4-alkanediols), are obtained as side products. The only additional compound formed is water which can be recycled in other reactions or disposed without having any negative ecological or economic impact. Consequently, it is possible to decrease the amount of side-product formation and allow for the gentle and sustainable preparation of high-purity 1-alkenes and thus highly pure 1,2-alkanediols. Also, non-reacted alcohol and intermediary formed alcohol are fully consumed during recirculation.

However, the present process allows for the targeted adjustment of the 1-alkene to 2-alkene and/or 3-alkene ratios and thus for the efficient adjustment of the composition of the final alkanediol product/composition as desired.

Preferably, the amount of 2-alkanes and/or 3-alkanes or other positional isomers other than the 1-alkene is below 5 % by weight referring to the total alkene product, preferably less than 3 % by weight.

In addition, the mild reaction conditions allow for a considerable increase in catalyst service life, which underlines the advantages and sustainability of the process according to the invention in terms of ecological and economic aspects.

In a second step b) the 1-alcohol is dehydrated to obtain at least one alkene having 5 to 15 carbon atoms, preferably 5 to 9 or further preferred 6 to 9 carbon atoms and even more preferred 7 or 8 carbon atoms, and most preferred 7 carbon atoms, wherein the at least one alkene having 5 to 15 carbon atoms, and preferably 5 to 9 or 6 to 9 carbon atoms and even more preferred 7 or 8 carbon atoms, is at least one olefin, selected from the group consisting of: α-, β- and/or γ-olefins, preferably a α-olefin having the corresponding chain length.

In order to prepare high purity 1,2-alkanediols, the as-obtained olefin is preferably a (high-purity) α-olefin. However, as indicated above, it is also possible to prepare a mixture of olefins varying in chain length. These different olefins themself can be α-, β- and/or γ-olefins.

Moreover, based on the reaction conditions it is possible to specifically adjust the ratios of the α-, β- and/or γ-olefins and thus the corresponding alkanediols.

Additionally, or alternatively, in step a) dialkylether is cleaved to form the corresponding 1-alcohols and/or 1-alkenes.

α-Olefins are alkenes having a double bond at the primary or alpha (α) position of the molecule (1-olefin/alkene). Accordingly, in a β-olefin the double bond is located between the second and third C-atom of the molecule (β-position: 2-olefin/alkene) while in γ-olefins the double bond is located between the third and fourth C-atom of the molecule (γ-position: 3-olefin/alkene).

The terms "olefin" and "alkene" are used synonymously. Also, the terms "α-olefin", "α-alkene", "1-olefin" and "1-alkene" are used synonymously. The same applies for the corresponding isomers "β-olefin", "β-alkene", "2-olefin" and "2-alkene" or "γ-olefin", "γ-alkene", "3-olefin" and "3-alkene".

As the product of the process according to the invention consist of or comprises a linear 1,2-alkanediol or a mixture of a linear 1,2-alkanediol and a 2,3-alkanediol and/or a 3,4-alkanediol in specific ratios wherein the content of the 2,3-alkanediol and/or the 3,4-alkanediol is low relative to the main product, i.e. the 1,2-alkanediol, the intermediate product of step b), i.e. the olefin compound can consist of or comprise a linear α-olefin or a mixture of a linear α-olefin with a β-olefin and/or a γ-olefin after the dehydration of the alcohol and/or cleavage of the dialkylether having the chain lengths specified above. Preferably, the as-obtained olefin is a high-purity α-olefin or a α-olefin with low β-olefin and/or γ-olefin contents in the specified ratios.

Therefore, in a preferred variant it is important to provide for a high-purity α-olefin having a chain length of 5 to 15 carbon atoms in step b) as intermediates in the preparation of high-purity 1,2-alkanediols having a chain length of 5 to 15 carbon atoms.

During the dehydration dialkylether might be formed and the product of step b) can additionally comprise unreacted educt, i.e. 1-alcohol or newly formed 1-alcohol based on the following reaction scheme originating from the cleavage of the dialkylether:

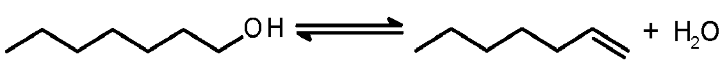

1-Heptanol 1-Heptane (α-olefin )

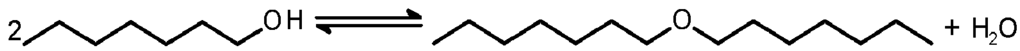

Diheptyl ether

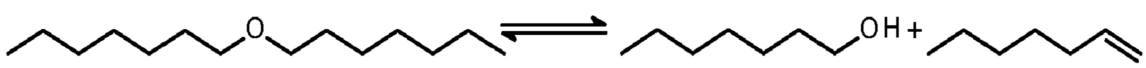

*Reaction scheme* (1): *Dehydration of 1-alcohol to the corresponding alkene and side product formation*

This alcohol can be recirculated together with the dialkylether formed in the process in order to efficiently reduce waste and simultaneously improve the balance of conversion efficiency, yield, purity and selectivity. Additionally, in cases wherein dialkylether is provided in step a) as starting material, step b) can also comprise non-reacted dialkylether, which is also recirculated. Therefore, in step b) dialkylether and 1-alcohol is recirculated.

Possible isomers of the intermediary formed 1-alkene according to the inventive process are exemplarily shown for 1-heptene in the following:

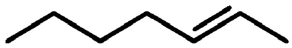
(E)-2-Heptene

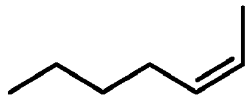
(Z)-2-Heptene

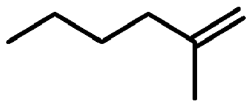
2-Methylhex-1-ene

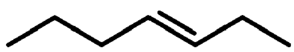
(E)-3-Heptene

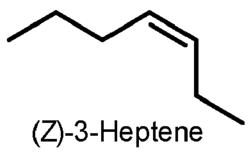
(Z)-3-Heptene

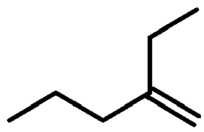
3-Methylenehexane

*Structural isomers* of *1-heptene*

With the present approach, it is possible to achieve a specific and low amount of said olefins as additional component or side products and thus of the corresponding diols within the final product, while allowing for a high yield of the particularly desired 1,2-alkanediols with excellent purity via the preparation of high-quality $\alpha$-olefins as intermediates in step b) or the corresponding isomeric (intermediate) compositions of at least two of the isomers.

Thereby, the dehydration and/or cleavage according to step b) of the process according to the invention is performed with recirculation of said dialkylether as well as 1-alcohol as specified above. Overall, this allows for a more efficient and sustainable approach towards a more ecological and economical preparation of 1,2-alkanediols with increased yields and purities at moderate reaction conditions while simultaneously allowing for a considerable reduction in waste products and costs. In addition, additional costs for waste treatment are more or less minimized.

Thereby it is sufficient to perform the dehydration at moderate temperatures ranging from 255 °C to 290 °C and using commercially available catalyst systems in order to achieve an ideal balance between selectivity, yield and purity of the 1,2-alkanediols by simultaneously reducing the amount of waste products and the energy and costs required for the reaction and for waste disposal.

However, preferably in the process according to the invention, the dehydration of step b) is performed at a temperature ranging from 260 °C to 280 °C and even more preferred from 270 °C to 280 °C. If the dehydration of step b) is performed within this range, an ideal balance of yield, purity and selectivity at moderate reaction conditions could be achieved. The relatively low reaction temperatures allow for the efficient preparation of $\alpha$-olefins and consequently of 1,2-alkanediols with lesser formation of $\beta$- and/or $\gamma$-olefins.

Advantageously, the dehydration is performed in a reactor with gaseous educts passing over the catalyst. Suitable reactors are for example fixed bed reactors, fluidized bed reactors, tubular reactors, and other suitable reactors allowing for intimate contact between the educt and the catalyst. Preferably, in the process according to the invention the reaction chamber used in step b), and in which the dehydration of the alcohols and/or cleavage of the dialkylether takes place, is a fixed bed reactor, preferably having at least one reaction zone equipped with a catalyst as specified above.

Preferably, the reaction chamber is a commercially available standard fixed-bed reactor as such a reactor is preferred due to its lower costs and facilitated handling. Alternatively, a so-called tube bundle reactor can be used.

In addition, the liquid hourly space velocity (LHSV) of the reactants over the catalyst within the reaction chamber for dehydration in step b) is preferably ranging from 0.5 to 10 1/h, even more preferred from 0.5 to 5 1/h or from 0.5 to 3.5 1/h, in order to achieve an efficient dehydration and/or cleavage. This allows for a mild conversion of the reactants due to reduced residence times of the reactants on the catalyst. This allows for an additional increase in the catalyst life cycle. If the LHSV of the reactants over the catalyst is within the ranges specified above, it is simultaneously possible

to additionally reduce the formation of β- and/or γ-olefins and thus of side products.

It was surprisingly found that it was possible to increase the purity and yield at moderate reaction conditions and achieve an ideal balance of yield, selectivity and/or purity in step b), and thus the overall product, by recirculating dialkylether and 1 -alcohol back into the reaction chamber and over the catalyst simultaneously allowing for a considerable reduction of waste and the use of moderate temperatures, both being beneficial from an ecological and economical point of view.

In addition, the recirculation promotes a gentler preparation of the desired 1,2-alkanediols at moderate conditions. The use of a nonmodified catalyst usually results in a reduction of the product selectivity and thus reduced purities and yield while low temperatures likewise result in a decreased conversion of the educts and thus in reduced yields and purities of the intermediate olefin compounds of the overall process described herein. However, on the other hand, low temperatures reduce the probability of intramolecular rearrangements of the double bonds and consequently the formation of 2-alkenes and 3-alkenes and thus 2,3-alkanediols and 3,4-alkanediols allowing for a high 1,2-alkanediol to 2,3-alkanediol and/or 3,4-alkanediol ratio, i.e. high purity 1-alkenes and 1,2-alkanediols, respectively. However, it was found that the recirculation of non-reacted dialkylether or intermediary formed dialkylether and/or non-reacted 1-alcohol or 1-alcohol side products allows for an improved balance between conversion, selectivity and yield, allowing for the ecological and efficient formation of 1-alkenes and 1,2-alkanediols with high purities, respectively.

Likewise, reduced residence times of the reactants at the catalyst cause lower conversion rates and thus yields, but on the other hand allow for a higher 1-alkene selectivity and purity. However, it was surprisingly found that the reduced conversion rates and yields can be efficiently compensated by recirculation in an eco-friendly manner at low costs.

Preferably, in the process according to the first aspect, the non-reacted or intermediary formed dialkylether and non-reacted and newly formed 1-alcohol are recirculated and combined with fresh educt, i.e. with fresh 1-alcohol and/or fresh dialkylether, preferably fresh 1-alcohol as explained above. Thereby, in a preferred variant in the recirculation, the ratio of freshly added 1-alcohol (i.e. alcohol that was not yet involved in the reaction) to the recycling compounds (i.e. non-reacted educt and dialkylether and alcohol formed in the preparation of the alkenes) ranges from 95 : 5 by volume (corresponding to low recycling rates, i.e. high amounts of fresh 1-alcohol are added) to 5 : 95 by volume (corresponding to high recycling rates, i.e. low amounts of fresh 1-alcohol are added). Preferably the ratio is between 80 : 20 and 20 : 80 in step b) of the process according to the invention. Preferably the ratio of the recycling compounds to the freshly added 1-alcohol is 30 : 70 to 50 : 50 by volume. This approach allows for an increased efficiency of the process and allows for the continuous preparation of the desired 1,2-alkanediol products via the preparation of high-quality α-olefins in a sustainable and gentle manner. Advantageously, the ratio of the recycling compounds to freshly added 1-alcohol is 40 (+/-10) : 60 (+/-10) by volume corresponding to a recirculation rate of 40 % +/- 10 % by volume relative to the total volume of the recycling compounds and freshly added 1-alcohol. This recirculation rate is most preferred in order to achieve the ideal balance between conversion, selectivity, yield and purity taking ecological and economical aspects into account.

Optionally, dialkylether can be selected as the sole or additional starting educt. Additionally, the corresponding dialkylether can be the only substance recirculated, if it is separated from the other compounds before recirculation.

Therefore, in a preferred variant of the inventive process, the dehydration and/or cleavage of step b) is performed as a continuous steady-state process, wherein the intermediary formed side products in the preparation of the alkenes and nonreacted educts (i.e. the recycling compounds) are continuously recirculated after simple separation of aqueous components and the product. Optionally, it is possible to add a distillation step before the recirculation for additional purification of the olefin-products.

However, also batchwise preparations are suitable.

In any case, optionally the collected olefin-products can be subjected to an additional distillation treatment allowing for the isolation of high-purity olefins, preferably α-olefins having 5 to 15 carbon atoms reaching purities up to 99 % or higher and allowing for the preparation of high-purity 1,2-alkanediols without the compelling requirement of additional purification steps.

Thereby, preferably the gaseous educt, i.e. the 1-alkanol is passed through the reactor comprising the catalyst at an internal temperature of 255 °C to 290 °C and preferably 270 °C to 280 °C and thus less than 280 °C.

Generally, for the endothermic process described herein, the required energy can be provided by various process approaches: (i) by external heating of the reaction reactor (tube-bundle reactor) by using a heat transfer medium such as oil, an electrical source, or any other external heat source; (ii) by usage of an adiabatic fixed bed reactor using an internal heat transfer medium such as $N_2$ or a $H_2O$ steam; and (iii) by usage of an adiabatic fixed bed reactor using a superheated alcohol (educt) steam in such a way, that the preferred reaction temperature is reached at the reactor outlet.

However, if the temperature for heating is in the range of 300 °C to 500 °C, high costs, an increase of the susceptibility to corrosion of the apparatus and an aggravation of the handling and control of the heat transfer media can be observed.

In addition, preferably a so-called purge gas is used which is usually an inert gas such as nitrogen. However, the use of such a gas is not decisive for the efficiency of the present invention and might be omitted. Generally, low

amounts of purge gas are preferred. The use of such purge gas has a diluting effect, wherein an increase in dilution results in a deterioration of the LHSVs, while low dilution rates equal better LHSV values. Consequently, a bigger cooling equipment would be required in order to collect the product of step b). Thus, alternatively, the process according to the invention can be performed without the use of gaseous $N_2$ at all.

If a purge gas is used, an additional dilution of the reactants can be achieved. By increasing the inert gas portion, the conversion efficiency can be increased while however, simultaneously resulting in a decreased $\alpha$-olefin purity and thus in an increased portion of the $\beta$-olefins and/or $\gamma$-olefins in the product of step b). Therefore, rather low dilutions are preferred.

Subsequently, the as-obtained intermediate gaseous product of step b) comprising the alkenes, possibly non-reacted alkanol and/or dialkylether as well as intermediary formed dialkylether or other side products are condensed. After separation of the aqueous phase the organic phase is preferably distilled in order to separate the desired alkenes and the remaining distillation bottoms are recirculated into the reaction chamber, and combined with fresh educt, preferably fresh alcohol in the ratios specified above, for further reaction. Preferably, at the end of the process, the as-obtained alkene-products are purified by additional distillation treatment.

This process could also be implemented as continuous process.

Preferably, the recirculation rate (or recycle rate) of the organic bottoms (recycling compounds) comprising dialkylether and 1-alcohol relative to the total volume of the organic bottoms and freshly added 1-alcohol is in the range of 20 % to 80 % by volume and preferably 30 % to 50 % by volume. The recirculation rate as used herein is defined as the ratio of the recirculated dialkylether and 1-alcohol to freshly added educt, i.e. 1-alcohol and/or dialkylether, but preferably 1-alcohol, by volume as indicated above. Most preferred, the recirculation rate is 40 % (+/- 10 %) by volume.

Thus, preferably, in each recycling cycle about 40 % (+/- 10 %) by volume of recycling compounds comprising dialkylether and 1-alcohol and about 60 % (+/- 10 %) by volume fresh 1-alcohol are fed into the reaction chamber. This corresponds to a recirculation rate of approximately 40 % (+/- 10 %) (i.e. a volume partition of 40 % (+/-10 %) of the recirculated compounds by volume relative to the total dosage).

Based on the experiments, it can be assumed, without being bound to this assumption, that the organic bottoms comprise approximately 40 % to 80 % by weight of dialkylether and 60 % to 20 % by weight of 1-alcohol. As the process described herein is a self-regulated process, it follows that the more dialkylether is formed in step b), the more dialkylether is recirculated and vice versa. This assumes that water has been removed. If water is still present the above would be adapted accordingly.

The above preferred recirculation ratios allow for a further reduction of the reaction temperature required and thus for milder reaction conditions as such, resulting in a more gently process and reducing the ecological end economic impact of the preparation process when compared to state-of-the-art processes. Moreover, no complex and thus costly additional modification of the catalyst or waste disposal is required. Nevertheless, it is possible to achieve an excellent product purity based on an improved balance of yield, selectivity and conversion while simultaneously considering all ecological and economic aspects, in particular a considerably reduced waste production.

Thereby, surprisingly the overall conversion rate of the 1-alcohol can be increased up to 100 % in total. Surprisingly, it was possible to find the optimal process condition from an economical point of view allowing for an excellent balance between selectivity and recycling by selecting a recirculation rate of about 40 % (+/- 10 %) by volume and simultaneously allowing for excellent product purities. When using an unmodified $Al_2O_3$ catalyst without recirculation, only a poor alkene purity of approximately 90 % would have been expected even at 100 % conversion, which is not sufficient for industrial applications. However, based on the inventive process it is possible to efficiently increase the product purity at high conversion rates through recycling.

However, also a comparable preparation of 1-alkenes based on the reaction of the corresponding dialkylether as only educt is possible, i.e., the reaction of pure dialkylether instead of the usage of alcohol compounds. In this case, the dialkylether is cleaved, in order to form an alcohol and the desired alkene compound. Subsequently, nonreacted ether, the formed alcohol and other by-products can be separated and optionally recirculated in order to achieve an ecological and economical process. Alternatively, a combination of 1-alcohol and dialkylether as educts can be used. However, preferably 1-alcohol is used as the educt.

Surprisingly, it was found that it is advantageous if the recirculation of the intermediate product is performed within the recirculation rates and temperature ranges specified above and that a highly efficient overall process especially in terms of costs, yield and product quality could be provided allowing for the ecologically and economically advantageous preparation of 1-alkenes and thus also 1,2-alkanediols with high purities. While it was possible to reduce the overall amount of waste products it was simultaneously possible to improve the balance of selectivity, purity and yield. Moreover, it is possible to achieve a high degree of alkene purity using commercially available catalysts and allowing for high catalyst service lives. Although the yield is decreasing due to the formation of dialkylether, this side product can be efficiently consumed and converted into high-quality 1-alkenes at low temperatures by recirculation for the subsequent conversion into 1,2-alkanediols, preferably in form of a steady state process. Thereby, the overall waste product is minimized while simultaneously allowing for an excellent purity and consequently an improved balance between yield,

purity and ecological and economical aspects.

Table 1 summarized the influence of the recirculation rate (recycling) of the distillation bottoms relative to the total feed as specified above on the conversion, selectivity, yield and α-olefin purity at moderate temperatures (< 290 °C). The term "distillation bottoms" refers to the intermediary formed side products such as the dialkylether or 1-alcohol and nonreacted educts which were separated from the product and aqueous components at the end of a reaction cycle by simple distillation and/or phase separation.

**Table** 1: Influence of the recirculation rate (recycling) in the conversion, selectivity, yield, and olefin purity (T = 274 °C +/-8 °C).

| **Recycling [%]** | **20** | **40** | **60** |
|---|---|---|---|
| **Temperature [°C]** | 282 | 274 | 266 |
| **Conversion [%]** | 92 | 86 | 82 |
| **Selectivity [%]** | 87 | 71 | 49 |
| **Yield [%]** | 80 | 61 | 40 |
| **α-Olefin purity [%]** | 96.1 | 96.9 | 97.6 |

It is possible to obtain the primarily desired 1-alkenes in purities of at least 95 % and up to 98 % or higher. A recirculation rate of about 50 % as defined above results in a α-olefin purity of about 97 % or higher and a recirculation rate of about 60 % allows for the preparation of α-olefins having a purity of about 98 %. However, these conditions are not economic due to low yields and selectivities as well as due to high recirculation rates. After additional distillation purities of 99 % or higher can be achieved. Regarding an ideal balance between conversion, selectivity, yield and purity, a recycling rate of approximately 40 % (+/-10%) by volume is preferred, e.g. optimizing the above, the recycling rate is preferably between 30 to 50 %, more preferably 40 % (+/-10%) by volume.

In addition, it could be observed that the balance between dialkylether formation and cleavage can be controlled by the volume partition of the recirculated distillation bottoms added to the fresh feed of e.g. 1-alcohol (i.e. the recirculation rate), the reaction temperature, dilution with purge gas, i.e. an inert gas serving as heat transfer and/or transport medium and thus the residence time or flow of the reactants as well as the pressure.

The adjustment of these parameters as indicated above allows for improved 1-alcohol conversion as well as high 1-alkene purities at moderate reaction conditions and thus an ideal balance between conversion and purity for an economical, sustainable, and gently process.

In a third step c) the at least one alkene obtained in step b), which is preferably a 1-alkene, is reacted to obtain a product comprising the corresponding 1,2-alkanediol (or the corresponding 2,3-alkanediol or the corresponding 3,4-alkkanediol) or a composition comprising or consisting of at least one alkanediol having 5 to 15 carbon atoms or a mixture of at least two alkanediols selected from the group consisting of: a 1,2-alkanediol, a 2,3-alkanediol and a 3,4-alkanediol, each independently having 5 to 15 carbon atoms as specified above, wherein in the product, the ratio of the 1,2-alkanediol to the 2,3-alkanediol is in the range of 90 : 10 to 99.9 : 0.1 and/or the ratio of the 1,2-alkanediol to the 3,4-alkanediol is in the range of 90 : 10 to 99.99 : 0.01 as specified above.

Thus, based on said process according to the first aspect, it is possible to obtain a composition comprising or consisting of at least two alkanediols, wherein the number of the carbon atoms of a first linear alkanediol and a second linear alkanediol and optionally a third linear alkanediol in the mixture is the same (homo combination). For example, the first linear alkanediol and the second linear alkanediol have a carbon chain of 7 carbon atoms, but the first linear alkanediol and the second linear alkanediol are different with regard to their constitutional isomerism, i.e. the positions of the OH-groups are different.

The purity of the as-obtained 1,2-alkanediols is generally comparable to the purity of the intermediary formed olefin compound and is 95 % or higher, preferably 96 % or higher, even more preferred 97 % or higher, especially preferred 98 % or higher and most preferred 99 % or higher. By additional, distillation of the products easily product purities of up to 99.9 % can be achieved.

The reaction of the alkenes to the corresponding alkanediols is known to the person skilled in the art and is based on any suitable technique known in the art. Preferably, the alkene is converted into the corresponding alkanediol by reaction with formic or acetic acid in the presence of hydrogen peroxide, and further treatment with an aqueous base solution. Optionally, the as-obtained product is additionally distilled to further reduce the amounts of 2,3- and/or 3,4-alkanediols.

Therefore, in a preferred variant of the process according to the invention, step c) is accomplished by reacting the at least one alkene (and preferably the 1-alkene) with formic acid and peroxide followed by subsequent reaction with

water and sodium hydroxide.

The alkenes obtained in step b) as well as the final alkanediols obtained in step c) of the inventive process each can be subject to an additional distillation step for further purification.

The purity of the as-obtained 1,2-alkanediols is 98 % or higher after distillation, even more preferred 99 % or higher. Alternatively, the ratio of the 1,2-alkanediol to the 2,3-alkanediol obtained by the inventive process, and more specifically in step c) of said process, is in the range of 90 : 10 to 99.9 : 0.1 and/or the ratio of the 1,2-alkanediol to the 3,4-alkanediol is in the range of 90 : 10 to 99.99 : 0.01. Preferably, the ratio of the 1,2-alkanediol to the 2,3-alkanediol is in the range of 95 : 5 to 99.9 : 0.1 and preferably from 97.5 : 2.5 to 99.9 : 0.1 and/or the ratio of the 1,2-alkanediol to the 3,4-alkanediol is in the range of 95 : 5 to 99.99 : 0.01 and preferably from 97.5 : 2.5 to 99.99 : 0.01.

Therefore, in a preferred variant of the inventive process, the process additionally comprises at least one distillation step after step b) and/or step c).

Preferably, after both steps a distillation is performed. In addition, it is advantageous if the distillation apparatus used after step b) is directly connected to the reaction chamber to allow a continuous process.

For example, a phase separator can be connected to the reaction chamber to separate organic components from aqueous components obtained in step b). Thereby, the collected organic phase is cooled down. In a subsequent distillation step the desired alkenes can be isolated form the collected organic phase by heating. Thereafter, the remaining distillation bottoms are recirculated into the reaction chamber for further conversion into the desired alkenes. Consequently, this approach is particularly suitable for a batch-based process.

Alternatively, the reaction chamber is connected to a distillation apparatus allowing for the direct recirculation of the distillation bottoms, i.e. the recycling compounds to the reactor allowing for a continuous process.

In a second aspect the invention relates to a composition comprising or consisting of at least two alkanediols selected from the group consisting of: a 1,2-alkanediol, a 2,3-alkanediol and a 3,4-alkanediol, each independently having from 5 to 15 carbon atoms, preferably from 6 to 9 carbon atoms and even more preferred from 7 to 8 carbon atoms, and most preferred 7 carbon atoms, wherein the ratio of the 1,2-alkanediol to the 2,3-alkanediol is in the range of 90 : 10 to 99.9 : 0.1 by weight and/or wherein the ratio of the 1,2-alkanediol to the 3,4-alkanediol is in the range of 90 : 10 to 99.99 : 0.01 by weight which is preferably obtainable through the process according to the invention. Preferably, the ratio of the 1,2-alkanediol to the 2,3-alkanediol is in the range of 95 : 5 to 99.9 : 0.1 and preferably from 97.5 : 2.5 to 99.9 : 0.1 by weight and/or the ratio of the 1,2-alkanediol to the 3,4-alkanediol is in the range of 95 : 5 to 99.99 : 0.01 and preferably from 97.5 : 2.5 to 99.99 : 0.01 by weight.

Preferably, the portion of the 1,2-alkanediols to the 2,3-alkanediols and/or 3,4-alkanediols is as high as possible within the above ranges.

According to a particularly preferred embodiment of the second aspect the composition, which is preferably obtained by the inventive process, comprises or consists of a 1,2-alkanediol as first linear alkanediol and a 2,3-alkanediol as second linear alkanediol, each independently having 5 to 15 carbon atoms, wherein the ratio of the 1,2-alkanediol to the 2,3-alkanediol is in the range of 90 : 10 to 99.9 : 0.1 by weight, and preferably in the range of 95 : 5 to 99.9 : 0.1 and even more preferred from 97.5 : 2.5 to 99.9 : 0.1 by weight.

Even more preferred, thereby, no 3,4-component is present at all or only a very low amount of the corresponding 3,4-component.

Thereby, the 1,2-alkanediol has from 5 to 15 carbon atoms and the 2,3-alkanediol has from 5 to 15 carbon atoms, wherein the number of the carbon atoms of the first and the second alkanediol is either the same (homo combination) or different (hetero combination).

Alternatively, the composition comprises or consists of a 1,2-alkanediol and a 3,4-alkanediol, each independently having 5 to 15 carbon atoms, wherein the ratio of the 1,2-alkanediol to the 3,4-alkanediol is in the range of 90 : 10 to 99.99 : 0.01 by weight, and preferably in the range of 95 : 5 to 99.99 : 00.1 and even more preferred from 97.5 : 2.5 to 99.99 : 0.01 by weight.

Furthermore, the composition can comprise or consist of a mixture of a 2,3-alkanediol and a 3,4-alkanediol.

According to another preferred embodiment, the composition comprises or consists of a 1,2-alkanediol, a 2,3-alkanediol and a 3,4-alkanediol, each independently having 5 to 15 carbon atoms, wherein the ratio of the 1,2-alkanediol to the 2,3-alkanediol is in the range of 90 : 10 to 99.9 : 0.1, and wherein the ratio of the 1,2-alkanediol to the 3,4-alkanediol is in the range of 90 : 10 to 99.99 : 0.01 by weight. Preferably, the ratio of the 1,2-alkanediol to the 2,3-alkanediol is in the range of 95 : 5 to 99.9 : 0.1 and preferably from 97.5 : 2.5 to 99.9 : 0.1 and/or the ratio of the 1,2-alkanediol to the 3,4-alkanediol is in the range of 95 : 5 to 99.99 : 0.01 and preferably from 97.5 : 2.5 to 99.99 : 0.01 by weight.

Referring to a further preferred embodiment the composition comprises or consists of a blend of alkanediols, wherein the 1,2-alkanediol and the 2,3-alkanediol and/or the 1,2-alkanediol and the 3,4-alkanediol and/or the 2,3-alkanediol and the 3,4-alkanediol have the same chain lengths.

Referring to another preferred embodiment the composition comprises or consists of a blend of alkanediols, wherein the 1,2-alkanediol and the 2,3-alkanediol and/or the 1,2-alkanediol and the 3,4-alkanediol and/or the 2,3-alkanediol and the 3,4-alkanediol have different chain lengths.

Thereby, preferably the composition comprises a blend of alkanediols, wherein the 1,2-alkanediol and the 2,3-alkanediol have different chain lengths (hetero combination).

According to an alternative embodiment said alkanediols are alkanediols each independently having chain lengths ranging from 5 to 9 carbon atoms, and preferably from 7 to 8 carbon atoms, provided that they have different chain lengths, preferably said mixture comprise a 1,2-alkanediol in combination with a 2,3-alkanediol. Such compositions may, for example, include any of the following mixtures/combinations:

- 1,2-pentanediol and 2,3-hexanediol;
- 1,2-pentanediol and 2,3-heptanediol;
- 1,2-pentanediol and 2,3-octanediol;
- 1,2-pentanediol and 2,3-nonanediol;
- 1,2-hexanediol and 2,3-pentanediol;
- 1,2-hexanediol and 2,3-heptanediol;
- 1,2-hexanediol and 2,3-octanediol;
- 1,2-hexanediol and 2,3-nonanediol;
- 1,2-heptanediol and 2,3-pentanediol;
- 1,2-heptanediol and 2,3-hexanediol;
- 1,2-heptanediol and 2,3-octanediol;
- 1,2-heptanediol and 2,3-nonanediol;
- 1,2-octanediol and 2,3-pentanediol;
- 1,2-octanediol and 2,3-hexanediol;
- 1,2-octanediol and 2,3-heptanediol;
- 1,2-octanediol and 2,3-nonanediol;
- 1,2-nonanediol and 2,3-pentanediol;
- 1,2-nonanediol and 2,3-hexanediol;
- 1,2-nonanediol and 2,3-heptanediol;
- 1,2-nonanediol and 2,3-octanediol;

wherein the ratio of the 1,2-alkanediol to the 2,3-alkanediol is in the range of 90 : 10 to 99.9 : 0.1 by weight, and preferably from 95 : 5 to 99.9 : 0.1, and even more preferred from 97.5 : 2.5 to 99.9 : 0.1 by weight.

According to an alternative and even more preferred embodiment the composition comprises or consist of a blend of alkanediols, wherein the 1,2-alkanediol and the 2,3-alkanediol and/or the 1,2-alkanediol and the 3,4-alkanediol and/or the 2,3-alkanediol and the 3,4-alkanediol have the same chain lengths (homo combination).

According to an even more preferred embodiment the composition comprises or consist of a blend of 1,2- and 2,3-alkanediols, wherein the 1,2-alkanediol and the 2,3-alkanediol have the same chain lengths. Preferably, no or only a very low amount of the corresponding 3,4-component is present.

Referring to this alternative and preferred embodiment, preferably, the alkanediols are alkanediols having chain lengths ranging from 5 to 9 carbon atoms, and preferably from 7 to 8 carbon atoms. Such compositions may, for example, include any of the following mixtures/combinations:

- 1,2-pentanediol and 2,3-pentanediol;
- 1,2-hexanediol and 2,3-hexanediol;
- 1,2-heptanediol and 2,3-heptanediol;
- 1,2-octanediol and 2,3-octanediol;
- 1,2-nonanediol and 2,3-nonanediol;

wherein the ratio of the 1,2-alkanediol to the 2,3-alkanediol is in the range of 90 : 10 to 99.9 : 0.1 by weight, and preferably from 95 : 5 to 99.9 : 0.1, and even more preferred from 97.5 : 2.5 to 99.9 : 0.1 by weight.

According to a particularly preferred variant thereof, the 1,2-alkanediol to the 2,3-alkanediol have a chain length of 7 carbon atoms, i.e. the composition according to the second aspect of the present invention preferably comprises or consists of a blend of 1,2-heptanediol and 2,3-heptanediol, showing the best properties in terms of odour, viscosity, antimicrobial efficiency, and stability against oxidation.

Thus, the composition can comprise or consist of a mixture of different alkanediols differing in chain lengths (hetero combination) or preferably alkanediols having the same chain lengths (homo combination) as specified above.

In order to achieve a combination of different chain lengths, the starting materials have to be selected accordingly based on their length.

According to another aspect of the present invention, it is possible to prepare compositions comprising a blend of two or more 1,2-alkanediols having different chain lengths based on the corresponding alkanes, or a blend of two or

more 2,3-alkanediols having different chain lengths, or a blend of two or more 3,4-alkanediols having different chain lengths based on the corresponding alkanes. Thereby, the blend can comprise two or more of the corresponding alkanediols, such as two different 1,2-alkanediols, three different 1,2-alkanediols, four different 1,2-alkanediols, ... each differing in their chain lengths, or two different 2,3-alkanediols, three different 2,3-alkandiols, four different 2,3-alkanediols, ... each differing in their chain lengths. In such hetero alkanediol mixtures the at least two alkanediols have a different number of carbon atoms (hetero combinations). Corresponding compositions of different 3,4-alkanediols are likewise possible.

In order to prepare such hetero combinations of 1,2-alkanediols, 2,3-alkanediols and/or 3,4-alkanediols the process according to the first aspect of the present invention has to be adapted accordingly.

Said adapted process for the preparation of alkanediol compositions/mixtures having 5 to 15 carbon atoms, wherein the single alkanediol components differ in their chain lengths, comprises the following steps:

a) providing at least one 1-alcohol having 5 to 15 carbon atoms and/or at least one dialkylether having 10 to 30 carbon atoms and a catalyst, preferably two or more 1-alcohols differing in their chain lengths and/or two or more dialkylethers differing in their chain lengths and/or asymmetric dialkylethers;

b) dehydrating the at least one 1-alcohol and/or cleavage of the at least one dialkylether to obtain at least one alkene having 5 to 15 carbon atoms, wherein the at least one alkene having 5 to 15 carbon atoms is at least one olefin selected from the group consisting of: $\alpha$-, $\beta$- and/or $\gamma$-olefins;

wherein the dehydration and/or cleavage is performed with recirculation of dialkylether and/or 1-alcohol; and wherein the dehydration and/or cleavage is performed at a temperature ranging from 255 °C to 290 °C;

c) reaction of the at least one alkene of step b) to obtain a product comprising or consisting of at least one alkanediol having 5 to 15 carbon atoms.

The product of step c) of said modified process is preferably a composition comprising or consisting of at least two 1,2-alkanediols differing in their chain lengths and/or a composition comprising or consisting of at least two 2,3-alkanediols differing in their chain lengths and/or a composition comprising or consisting at least two 3,4-alkanediols differing in their chain lengths, wherein said alkanediols each independently have from 5 to 15 carbon atoms. Preferably said product is a composition comprising or consisting of two 1,2-alkanediols differing in their chain lengths and/or a product comprising at least two 2,3-alkanediols differing in their chain lengths. Such preferred hetero combinations are thus:

- a mixture of two or more 1,2-alkanediols which differ in their chain lengths (hetero combination),
- a mixture of two or more 2,3-alkanediols which differ in their chain lengths (hetero combination), or
- a mixture of two or more 3,4-alkanediols which differ in their chain lengths (hetero combination).

Consequently, the present invention further relates to said compositions obtained or obtainable by the modified inventive process and their use for the preparation of different consumer products as well as said consumer products as such. Such products may amongst other include for example any of the following mixtures/combinations:

- 1,2-pentanediol and 1,2-hexanediol;
- 1,2-pentanediol and 1,2-heptanediol;
- 1,2-pentanediol and 1,2-octanediol;
- 1,2-pentanediol and 1,2-nonanediol;
- 1,2-pentanediol and 1,2-decanediol;
- 1,2-hexanediol and 1,2-pentanediol;
- 1,2-hexanediol and 1,2-heptanediol;
- 1,2-hexanediol and 1,2-octanediol;
- 1,2-hexanediol and 1,2-nonanediol;
- 1,2-hexanediol and 1,2-decanediol;
- 1,2-heptanediol and 1,2-pentanediol;
- 1,2-heptanediol and 1,2-hexanediol;
- 1,2-heptanediol and 1,2-octanediol;
- 1,2-heptanediol and 1,2-nonanediol;
- 1,2-heptanediol and 1,2-decanediol;
- 1,2-octanediol and 1,2-pentanediol;
- 1,2-octanediol and 1,2-hexanediol;

- 1,2-octanediol and 1,2-heptanediol;
- 1,2-octanediol and 1,2-nonanediol;
- 1,2-octanediol and 1,2-decanediol;
- 1,2-nonanediol and 1,2-pentanediol;
- 1,2-nonanediol and 1,2-hexanediol;
- 1,2-nonanediol and 1,2-heptanediol;
- 1,2-nonanediol and 1,2-octanediol;
- 1,2-nonanediol and 1,2-decanediol;

The different combinations of the corresponding at least two 2,3-alkanediols as well as the corresponding 3,4-alkanediol can be formulated correspondingly.

Preferably the two or more different 1,2-alkanediols or the two or more different 2,3-alkanediols or the two or more different 3,4-alkanediols, respectively, are selected from alkanediols having chain lengths of 5 to 9 carbon atoms.

These specified hetero alkanediol mixtures according to this aspect of the present invention, show a synergistically intensified action, compared to their corresponding single 1,2-alkanediol or 2,3-alkanediol substances as will become apparent form the experimental section.

Furthermore, according to another variant of the present invention, the compositions comprising or consisting of at least two 1,2-alkanediols differing in their chain lengths can further comprise minor amounts of the corresponding 2,3-alkanediols. Thereby, the ratio of the respective 1,2-alkanediol to the corresponding 2,3-alkanediol is in the range of 90 : 10 to 99.9 : 0.1 by weight, and preferably from 95 : 5 to 99.9 : 0.1, and even more preferred from 97.5 : 2.5 to 99.9 : 0.1 by weight.

Additionally, the present invention relates to the composition comprising or consisting of at least two 1,2-alkanediols differing in their chain lengths and/or a composition comprising or consisting at least two 2,3-alkanediols differing in their chain lengths, wherein said alkanediols each independently have from 5 to 15 carbon atoms obtained or obtainable by the modified process specified above.

Another aspect of the invention relates to the use of the composition according to the invention for the preparation of a variety of consumer products such as cosmetics, personal care products, in particular skin cleaning products, shampoos, rinse-off conditioners, deodorants, antiperspirants, body lotions, homecare products, textile care products, household products, in particular liquid detergents, all-purpose cleaners, laundry and cleaning agents, fabric softeners, scent boosters, fragrance enhancers and pharmaceutical compositions and the like.

Finally, another aspect relates to consumer products as such, such as cosmetics, personal care products, in particular skin cleaning products, shampoos, rinse-off conditioners, deodorants, antiperspirants, body lotions, homecare products, textile care products, household products, in particular liquid detergents, all-purpose cleaners, laundry and cleaning agents, fabric softeners, scent boosters, fragrance enhancers and pharmaceutical compositions comprising the composition according to the invention.

However, the consumer product described herein may comprise the inventive composition, wherein alkanediols having different chain lengths may be present as well as other active ingredients.

In the consumer products, the 1,2-alkanediol and/or 2,3-alkanediol are comprised in the alkanediol mixture preferably in a ratio ranging from 95 : 5 to 99.9 : 0.1.

Preferably, in the consumer products, the 1,2-alkanediol and 2,3-alkanediol are comprised in the alkanediol mixture preferably in a ratio of $\geq$ 95 : $\leq$ 5, more preferred in a ratio of $\geq$ 96 : $\leq$ 4; still more preferred in a ratio of $\geq$ 97 : $\leq$ 3, and most preferred in a ratio of $\geq$ 98 : $\leq$ 2 by weight.

Even more preferred, no or only traces of the corresponding 3,4-alkanediols are present within said alkanediol mixtures.

In the context of the present text, the terms "1,2-diol", "2,3-diol" or "3,4-diol" includes both the corresponding S-configured enantiomers and also the R-enantiomers well as arbitrary mixtures of these S- and R-configured enantiomers, i.e. mixtures of racemates of the respective diols.

## Experimental

General procedure:

The following preparations of bio-alkanediols, especially 1,2-alkanediols and in particular 1,2-heptanediol, have been performed in a reactor which is made of a 100 cm long steel tube having an inner diameter of 14 mm. Heating was performed by an electrical heating mantle which has three temperature zones: a preheating zone being 30 cm in length which is filled with an inert carborundum filling; a subsequent 50 cm long zone forming the catalytic fixed-bed and consisting of solid catalyst particles being arranged as a so-called fixed bed in the reactor thus forming the reaction zone or reaction chamber; followed by a zone being 20 cm long filled with inert carborundum material (vertical arrangement

from top to bottom). The three temperature zones are individually controlled. When reference is made to the temperature of the process, it is referred to the internal temperature in the middle zone forming the catalytic fixed-bed, i.e. the actual reaction zone where the dehydration and/or cleavage process takes place.

The catalyst bed is preheated and as soon as the desired temperature is reached the reactants are continuously pumped on top of the evaporation zone at rates of 0.5 ml/min to 10 ml/min and preferably 0.5 ml/min to 2.5 ml/min.

Liquid reagents are dosed with HPLC pumps and gaseous reagents are metered with the aid of mass flow controllers. Both, gaseous and liquid reagents, enter the reactor from the top of the vertical arrangement. The liquids flow along the first 30 cm of inert filling (preheating zone) due to gravity until complete evaporation. Progress is monitored by an internal temperature control. Thereby, the contact of the liquid with the catalyst filling should be avoided, as this could accelerate the deactivation of the catalyst.

The as-obtained gaseous educts enter the 50 cm catalyst zone. Due to the endothermic nature of the dehydration reaction a distinct temperature profile along the catalyst bed can be followed by an internal temperature control. In addition, a temperature profile across the catalyst bed might be observed, too.

Subsequently, the gaseous products pass the third zone with inert filling. Purpose of the last temperature zone is to avoid thermal loss and to achieve an isothermal reaction zone.

Finally, the gaseous products are condensed. Eventually non-condensable gaseous products are vented. The collected liquid products settle into an aqueous phase and an organic phase containing the desired alkenes which might be separated using simple phase separation methods known in the art. Simple distillation of the organic product phase finally yields the pure desired alkenes, preferably the 1-alkenes which are subsequently converted into 1,2-alkanediols according to step c) of the process. Additionally, the organic phase might be dried with a drying agent before distillation.

Subsequently, the as-obtained alkenes are converted to the corresponding alkanediols using common techniques.

Calculation of the 1-alkene fraction purity, conversion, selectivity and yield as well as liquid hourly space velocity:

In the following the term "heptene" refers to "1-heptene" while the term "isomers" refers to the structural isomers of the 1 -heptene as exemplarily shown above and "heptanol" refers to "1-heptanol".

The chemical purity of the 1-heptene fraction (relative to its mole number is defined as follows:

$$\text{Purity (heptene)} = \frac{\text{heptene}}{\text{heptene} + \text{isomers}}$$

*Equation (1): Purity of 1-alkene*

The molar conversion of the educt (i.e. 1-heptanol), selectivity and yield are defined as follows:

$$\text{Conversion} = \frac{2 \cdot \text{diheptyl ether} + \text{heptene} + \text{isomers}}{\text{heptanol} + 2 \cdot \text{diheptyl ether} + \text{heptene} + \text{isomers}}$$

*Equation (2): Molar conversion rate of 1-alcohol (= ratio of how much 1-alcohol has reacted)*

$$\text{Selectivity} = \frac{\text{heptene}}{2 \cdot \text{diheptyl ether} + \text{heptene} + \text{isomers}}$$

*Equation (3): Molar selectivity (= ratio of how much 1-alkene was formed in ratio to the other products)*

$$\text{Yield} = \frac{\text{heptene}}{\text{heptanol} + 2 \cdot \text{diheptyl ether} + \text{heptene} + \text{isomers}}$$

*Equation (4): Molar yield (= ratio of how much 1-alkene was formed)*

The liquid hourly space velocity (LHSV) is defined according to equation (5), wherein V(educt) represents the volumetric flow rate of the reactants entering the catalyst fixed bed and V(reactor) is defined as the volume of the catalyst fixed bed itself:

$$\text{LHSV} = \frac{\text{V(educt)}}{\text{V(reactor)} \cdot \text{time}}$$

*Equation (5): LHSV*

Example 1: Preparation of bio-alkenes based on the continuous addition of 1-alcohol - Temperature effect:

Bio-alkenes based on the continuous addition of pure 1-alcohol according to Example 1 were prepared as indicated above and based on the following reaction conditions:

| | |
|---|---|
| 1-Heptanol | 2.5 ml/min |
| $N_2$ | 8 $ml_n$/min |
| Fixed-bed | 14 mm ID x 500 mm (77.0 ml) |
| Catalyst | 63.0 g Alumina spheres (1.0 mm) from Sasol |

Thereby nitrogen gas serves as carrier gas and optionally as heat transfer medium.

According to the product information the catalyst used in the experiment, $Al_2O_3$, has a packed bulk density of 740 to 820 g/l, a surface area ranging from 150 to 170 $m^2$/g and a pore volume of at least 0.45 ml/g.

The catalyst bed was preheated and as soon as the desired temperature was reached 1-heptanol was continuously pumped on top of the evaporation zone at a rate of 2.5 ml/min. After two hours a steady state was achieved, and the corresponding temperature noted as well as a GC sample taken. Subsequently, the temperature was raised step by step as indicated in Table 2 and different curves showing the temperature dependence of the product composition, conversion, selectivity, yield and 1-heptene fraction purity are obtained (Figures 1A to 1C).

In this and the following examples the corresponding conversion rate, selectivity, yield and purity were calculated based on the equations specified above.

This example serves as control experiment.

Table 2 shows the corresponding product composition by weight in dependence of the temperature.

**Table 2:** Corresponding product composition by weight in dependence of the temperature.

| Temp. [°C] / Comp. [wt.-%] | 264 | 267 | 270 | 272 | 276 | 278 | 280 | 285 | 288 | 290 | 294 | 298 | 304 |
|---|---|---|---|---|---|---|---|---|---|---|---|---|---|
| 1-Heptene | 30.9 | 39.6 | 47.8 | 53.4 | 61.9 | 67.8 | 71.5 | 83.7 | 86.6 | 88.7 | 91.6 | 91.4 | 83.7 |

| | | | | | | | | | | | | | |
|---|---|---|---|---|---|---|---|---|---|---|---|---|---|
| Heptene Isomers | 0.7 | 1.0 | 1.4 | 1.6 | 2.1 | 2.5 | 2.7 | 3.8 | 4.2 | 4.6 | 5.6 | 7.3 | 16.3 |
| 1-Heptanol | 21.1 | 19.6 | 17.8 | 16.4 | 14.7 | 13.2 | 12.3 | 7.4 | 5.8 | 4.4 | 1.7 | 0.0 | 0.0 |
| Diheptyl Ether | 46.6 | 39.0 | 32.4 | 27.8 | 20.4 | 15.7 | 12.7 | 4.3 | 2.5 | 1.5 | 0.2 | 0.0 | 0.0 |
| Unknown | 0.7 | 0.7 | 0.7 | 0.8 | 0.9 | 0.7 | 0.8 | 0.8 | 0.9 | 0.8 | 0.9 | 1.2 | 0.9 |
| Conversion, selectivity, yield and purity of 1-heptene [%] | | | | | | | | | | | | | |
| Conversion | 80.7 | 82.1 | 84.0 | 85.3 | 86.9 | 88.3 | 89.2 | 93.6 | 95.0 | 96.2 | 98.5 | 100 | 100 |
| Selectivity | 41.6 | 51.9 | 60.6 | 66.3 | 74.9 | 80.1 | 83.3 | 91.5 | 93.0 | 93.7 | 94.1 | 92.6 | 83.7 |
| Yield | 33.6 | 42.6 | 50.9 | 56.6 | 65.1 | 70.7 | 74.3 | 85.7 | 88.4 | 90.2 | 92.7 | 92.6 | 83.7 |
| Purity | 97.8 | 97.5 | 97.2 | 97.1 | 96.7 | 96.4 | 96.4 | 95.7 | 95.4 | 95.1 | 94.2 | 92.6 | 83.7 |

At temperatures of 298 °C and higher a complete conversion of 1-heptanol is achieved. At 298 °C, however, the purity of 1-heptene is only 91.1 %, which is too low for industrial applications. On the other hand, the 1-heptene purity at 280 °C is excellent (96.4 %), but the 1-heptene yield reaches only 74.3 %, which is due to high selectivity losses caused by the formation of diheptyl ether as a by-product according to the reaction scheme shown above. Such a yield is too low for industrial application. Therefore, there is great interest in creating added value from the by-product diheptyl ether, which is demonstrated by the following examples.

Moreover, it can be concluded that full conversion does not result in the required and thus desired purities of the $\alpha$-olefin. In addition, it can be observed that an increase in temperature results in a decrease of the $\alpha$-olefin purity, especially for temperatures higher than 290 °C.

**Example 2:** Preparation of bio-alkenes based on the continuous addition of 1-alcohol and diheptyl ether - Influence of the diheptyl ether dosage:

Bio-alkenes based on the continuous addition of pure 1-alcohol and diheptyl ether according to Example 2 were prepared as indicated above and based on the following reaction conditions:

| | |
|---|---|
| Dosage | 2.5 ml/min (sum of 1-heptanol and diheptyl ether) |
| $N_2$ | 8 $ml_n$/min |
| Fixed-bed | 14 mm ID x 500 mm (77.0 ml) |
| Catalyst | 63.0 g Alumina spheres (1.0 mm) from Sasol |

The catalyst bed was preheated and as soon as the desired temperature was reached 1-heptanol was continuously pumped on top of the evaporation zone at a rate of 2.5 ml/min. After two hours steady state was achieved, and a GC sample taken. In the same way an increasing share of diheptyl ether was dosed while keeping the total volume flow constant at 2.5 ml/min.

At 20 °C 1-heptanol has a density of 0.819 g/ml and diheptyl ether has a density of 0.801 g/ml. Considering these small differences in density 10 % by volume correspond to 9.8 % by weight of diheptyl ether and 20 % by volume corresponding to 19.6 % by weight of diheptyl ether.

Table 3 shows the corresponding product composition by weight in dependence of the temperature and diheptyl ether dosage (proof-of-concept).

**Table 3:** Corresponding product composition by weight in dependence of the temperature and diheptyl ether dosage.

| Diheptyl Ether Dosage [vol.-%] | 0 | 0 | 10 | 10 | 20 | 20 |
|---|---|---|---|---|---|---|
| | Increasing diheptyl ether dosage → | | | | | |
| Comp. [wt.-%] \ Temp. [°C] | 273 | 283 | 273 | 283 | 273 | 283 |
| 1-Heptene | 56.7 | 80.0 | 56.3 | 76.3 | 63.3 | 80.4 |
| Heptene Isomers | 1.8 | 3.5 | 1.8 | 3.0 | 2.1 | 3.4 |
| 1-Heptanol | 16.1 | 9.5 | 15.8 | 10.8 | 13.9 | 8.9 |
| Diheptyl Ether | 24.7 | 7.0 | 26.2 | 9.8 | 20.6 | 7.2 |
| Unknown | 0.8 | 0.8 | 1.5 | 0.7 | 0.9 | 0.7 |
| Water | 11.4 | 13.7 | 10.6 | 12.6 | 10.6 | 12.3 |
| Conversion [%] | 85.6 | 91.8 | 86.0 | 90.7 | 87.8 | 92.4 |
| Selectivity [%] | 70.0 | 89.0 | 68.6 | 86.5 | 75.1 | 88.9 |
| Yield [%] | 59.9 | 81.7 | 59.0 | 78.4 | 65.9 | 82.1 |
| Purity [%] | 97.0 | 95.8 | 97.0 | 96.2 | 96.7 | 95.9 |

This example demonstrates that by small variation of the reaction temperature it is possible to find conditions at which either more diheptyl ether is formed or at which diheptyl ether is cleaved into additional 1-heptene value product. However, if solely 1-heptanol is used as feed always some diheptyl ether is formed as by-product. This means that diheptyl ether recirculation is mandatory to achieve an economically feasible process and to reduce the overall amount of waste products.

As expected, increasing diheptyl ether partition, i.e. an increasing relative diheptyl ether dosage is resulting in a reduced formation of reaction water.

In addition, it can be concluded that at 273 °C more diheptyl ether is formed than added, while at higher temperatures (283 °C) more diheptyl ether is consumed than was initially added.

Moreover, it is expected that the same effect can be observed when the dosing speed and thus the residence time of the reactants over the catalyst is varied.

**Example 3:** Preparation of bio-alkenes based on the continuous addition of 1-alcohol and dilution with nitrogen gas - Influence of the dilution of the reactants:

Bio-alkenes based on the continuous addition of pure 1-alcohol according to Example 3 were prepared as indicated above and based on the following reaction conditions and under increasing dilution with gaseous nitrogen:

| | |
|---|---|
| Fixed-bed | 14 mm ID x 500 mm (77.0 ml) |
| Catalyst | 63.0 g Alumina spheres (1.0 mm) from Sasol |

So far as standard conditions a 1-heptanol flow of 2.5 ml/min (17.62 mmol/min) and a $N_2$ flow of 8 $ml_n$/min (0.35 mmol/min) were chosen. Such small amounts of $N_2$ inert gas were only used to avoid a back mixing of small amounts of $O_2$ in the reaction zone by diffusion during the reaction and to avoid flooding of the still hot reaction zone while cooling down the reactor.

However, larger amounts of an inert gas such as $N_2$ may be used as diluent and heat transfer medium. Since the dehydration takes place with an increase in the mole number, a constant total flow for 1-heptanol, $H_2O$ and $N_2$ of 35.61 mmol/min was calculated. This is based on the described standard conditions that sum up to 2 times 17.62 mmol/min of 1-heptanol plus 0.35 mmol/min $N_2$. This ensures that the residence time of the reactants over the catalyst is kept constant and that only the $N_2$ inert gas dilution is varied during the experiment.

The temperature in all experiments according to Example 3 was kept constant at approximately 278 °C (+/- 4 °C).

Table 4 shows the corresponding product composition by weight in dependence of the dilution with gaseous $N_2$.

**Table 4:** Corresponding product composition by weight in dependence of the dilution with gaseous $N_2$.

| 1-Heptanol [ml/min] | 2.50 | 2.17 | 1.68 | 1.01 |
|---|---|---|---|---|
| $N_2$ [$ml_n$/min] | 8 | 114 | 266 | 479 |
| $N_2$ [mol.-%] | 2 | 25 | 50 | 75 |
| | Increasing dilution of the reactants with $N_2$ → | | | |
| Temp. [°C] / Comp. [wt.-%] | 276 | 276 | 278 | 282 |
| 1-Heptene | 61.9 | 77.7 | 92.0 | 86.0 |
| Heptene Isomers | 2.1 | 3.2 | 5.6 | 13.0 |
| 1-Heptanol | 14.7 | 9.9 | 1.5 | 0.0 |
| Diheptyl Ether | 20.4 | 8.4 | 0.2 | 0.1 |
| Unknown | 0.9 | 0.7 | 0.7 | 0.9 |
| Conversion [%] | 86.9 | 91.3 | 98.8 | 100 |
| Selectivity [%] | 74.9 | 87.7 | 94.1 | 86.8 |
| Yield [%] | 65.1 | 80.1 | 92.9 | 86.8 |
| Purity [%] | 96.7 | 96.0 | 94.3 | 86.9 |

These results show that an increasing portion of $N_2$ inert gas dilution is leading to higher conversion rates, but simultaneously to decreasing 1-heptene purities. In addition, it was shown that gaseous $N_2$ can suitably be used as internal heat transfer medium for adiabatic process alternatives.

Furthermore, it was observed that the dilution rate influences the formation of dialkylether. However, as indicated above, simultaneously the purity of the product decreases and an increased portion of the isomeric forms of the alkene are formed.

In aiming to reduce the content of isomeric forms, however, lower dilution rates are preferred allowing for the preparation of high quality 1-alkenes and thus the corresponding 1,2-alkanediols. It was now found that the reduced yield can be efficiently compensated by a recycling process as specified herein allowing for an improved balance of yield and purity while simultaneously reducing the overall amount of waste products. Consequently, in the following examples according to the invention preferably a low portion of $N_2$ is chosen corresponding to a low dilution.

In addition, it is expected that a pressure variation should have the same effect as the dilution with inert gas.

**Example 4:** Preparation of bio-alkenes based on the partial conversion of 1-alcohol - Influence of partial conversion:

Bio-alkenes based on the partial conversion of 1-alcohol according to Example 4 were prepared as indicated above and based on the following reaction conditions:

| | |
|---|---|
| 1-Heptanol | 2.5 ml/min |
| $N_2$ | 8 $ml_n$/min |
| Fixed-bed | 14 mm ID x 500 mm (77.0 ml) |
| Catalyst | 63.0 g Alumina spheres (1.0 mm) from Sasol |

The catalyst bed was preheated and as soon as the desired temperature was reached 1-heptanol was continuously pumped on top of the evaporation zone at a rate of 2.5 ml/min. After one hour a steady state was achieved with a temperature of 275 °C inside the catalyst bed. Thereafter, 2906.3 g 1-heptanol were added and 2893.8 g of the product collected within 24 hours. From the as-obtained product 353.8 g of a water layer were separated and the remaining 2539.0 g organic phase purified by distillation at 450 mmbar using a short 30 cm packed column equipped with a column

head.

In summary, the mass reaction balance was as follows:

| | |
|---|---|
| Water: | 353.8 g |
| 1-Heptene, pure: | 1511.5 g |
| 1-Heptene for re-distillation: | 129.6 g (middle run fraction) |
| Distillation bottoms: | 878.1 g |
| Loss: | 19.8 g |
| Σ g | 2892.8 g |

Table 5 shows the corresponding composition of the as-obtained product after removal of water, the isolated 1-heptene product, the composition of 1-heptene obtained in a distillation middle run fraction which is to be re-distilled (to avoid waste and product losses) and finally the composition of the remaining distillation bottoms after removal of the 1-heptene product.

Table 5: Corresponding product composition by weight.

| Compound [wt.-%] | Product as-obtained | Isolated 1-Heptene | 1-Heptene for re-distillation (middle run fraction) | Remaining bottoms available for recirculation |
|---|---|---|---|---|
| 1-Heptene | 61.0 | 96.5 | 90.6 | 0 |
| Heptene Isomers | 2.2 | 3.4 | 5.8 | 0 |
| 1-Heptanol | 15.2 | 0 | 1.3 | 42.8 |
| Diheptyl Ether | 20.8 | 0 | 0.2 | 55.5 |
| Unknown | 0.7 | 0.1 | 2.0 | 1.7 |
| Compound [g] | | | | |
| 1-Heptene | 1548.8 | 1458.7 | 117.4 | 0 |
| Heptene Isomers | 57.0 | 50.8 | 7.5 | 0.2 |
| 1-Heptanol | 386.0 | 0.3 | 1.7 | 376.0 |
| Diheptyl Ether | 529.2 | 0.3 | 0.3 | 486.9 |
| Unknown | 17.9 | 1.4 | 2.6 | 14.7 |
| Σ [g] | 2539.0 | 1511.5 | 129.6 | \| 878.1 |
| Conversion [%] | 86.5 | - | - | - |
| Selectivity [%] | 74.1 | - | - | - |
| Yield [%] | 64.1 | - | - | - |
| Purity [%] | 96.4 | 96.6 | 94.0 | - |

The GC product analysis indicates 86.5 % of 1-heptanol conversion, 74.1 % 1-heptene selectivity and 64.1 % 1-heptene yield. Losses can be mainly attributed to the formation of diheptyl ether and to the less extend formation of heptene isomers. Surprisingly, a simple distillation is already leading to 1511.5 g of a 1-heptene fraction having an excellent purity of 96.4 %. Referring to a 2906.3 g (25.011 mol) 1-heptanol feed this corresponds to an isolated yield of 1-heptene of 59.4 % (14.856 mol).

In addition, 129.6 g of a small middle run fraction containing further 4.8 % of additional 1-heptene (1.196 mol) crude yield for further isolation via re-distillation were obtained. The 878.1 g distillation bottoms contain mainly unwanted

diheptyl ether by-product and to less extend unconverted 1-heptanol. This means than an 18.2 % diheptyl ether (2.271 mol) crude yield was obtained. However, 353.8 g (19.639 mol) of water were formed corresponding to a yield of 78.5 %.

**Example 5:** Preparation of bio-alkenes based on the continuous addition of 1-alcohol and distillation bottoms - Recycling process according to the invention:

Bio-alkenes based on the dehydration of 1-alcohol according to Example 5 were prepared as indicated above and based on the continuous addition of 1-alcohol and distillation bottoms (recycling process according to the invention) and the following reaction conditions:

| | |
|---|---|
| Distillation Bottoms | 42.8 % 1-heptanol, 55.5 % diheptyl ether and 1.7 % of unknown residues (see Example 4) |
| Dosage | 2.5 ml/min of 1-heptanol and distillation residues/bottoms in total |
| $N_2$ | 8 $ml_n$/min |
| Fixed-bed | 14 mm ID x 500 mm (77.0 ml) |
| Catalyst | 63.0 g Alumina spheres (1.0 mm) from Sasol |

The catalyst bed was preheated and as soon as the desired temperature was reached, fresh 1-heptanol was continuously pumped on top of the evaporation zone at a rate of 2.5 ml/min. After two hours a steady state was reached, and a GC sample collected. In the same way, an increasing share of distillation bottoms (from Example 4) was dosed while keeping the total volume flow constant at 2.5 ml/min.

At 20 °C 1-heptanol has a density of 0.819 g/ml and the density of the distillation bottoms was 0.809 g/ml. For reference, 10 % by volume distillation bottoms correspond to 5.6 % by weight of diheptyl ether (corresponding to a recirculation rate of 10 %) while 20 % by volume distillation bottoms correspond to 11.2 % by weight of diheptyl ether (corresponding to a recirculation rate of 20 %) and 30 % by volume distillation bottoms correspond to 16.8 % by weight of diheptyl ether (corresponding to a recirculation rate of 30 %), 40 % correspond to 22.4 % by weight of diheptyl ether (corresponding to a recirculation rate of 40 %) and 50 % correspond to 28.1 % by weight of diheptyl ether (corresponding to a recirculation rate of 50 %).

The temperature in all experiments according to Example 5 with increasing dosage of recirculation bottoms was kept constant at approximately 275 °C (+/- 2 °C).

Table 6 shows the corresponding product composition by weight in dependence of the addition of distillation bottoms.

**Table 6:** Corresponding product composition by weight in dependence of the addition of distillation bottoms.

| Distillation Bottoms Dosage [vol.-%] | 0 | 10 | 20 | 30 | 40 | 50 |
|---|---|---|---|---|---|---|
| Temp. [°C] / Comp. [wt.-%] | 274 | 275 | 275 | 276 | 276 | 277 |
| 1-Heptene | 66.9 | 63.6 | 61.1 | 60.4 | 58.7 | 57.2 |
| Heptene Isomers | 2.6 | 2.2 | 2.0 | 2.0 | 2.0 | 1.9 |
| 1-Heptanol | 14.1 | 14.4 | 14.7 | 14.1 | 14.2 | 13.6 |
| Diheptyl Ether | 15.8 | 18.9 | 21.3 | 22.2 | 23.8 | 25.9 |
| Unknown | 0.6 | 0.8 | 1.0 | 1.4 | 1.2 | 1.5 |
| Water | 12.4 | 11.7 | 11.0 | 10.6 | 10.0 | 9.5 |
| Conversion [%] | 87.5 | 87.3 | 86.9 | 87.4 | 87.3 | 87.8 |
| Selectivity [%] | 79.7 | 76.5 | 74.0 | 73.0 | 71.1 | 69.1 |
| Yield [%] | 69.8 | 66.7 | 64.3 | 63.8 | 62.1 | 60.7 |
| Purity [%] | 96.3 | 96.6 | 96.8 | 96.8 | 96.7 | 96.8 |

This example demonstrates that by increasing the dosage of the distillation bottoms a certain point is achieved at which more diheptyl ether is cleaved than formed. This point is reached at a dosage of 40 to 50 % by volume of

distillation bottoms (corresponding to a recirculation rate between 40 % and 50 % by volume as specified above). If the formed reaction water is taken into account, it can be concluded that this point is located between a dosage of 30 % to 40 % by volume of distillation bottoms, i.e., a recirculation rate of 30 to 40 % by volume. Accordingly, a dosage of 30 % to 40 % by volume of distillation bottoms is advantageous for a waste reduced production process with high conversion rates and excellent product purities.

It should be noted, that in this example the distillation bottoms were not additionally purified before recycling.

Nevertheless, it was possible to obtain the desired 1-alkene, i.e. 1-heptene, having a high chemical purity.

In addition, it was observed that recycling of distillation bottoms allows for a further decrease of the required temperature to temperatures below 280 °C and approximately to 275 °C based on the reaction of the dialkylether to the corresponding 1-alcohol and 1-alkene. Comparably low reaction temperatures for the efficient preparation of 1-alkenes are not known from the state of the art.

This allows for mild reaction conditions, which allows for the further reduction of costs and increased equipment lifetimes. Based on the low temperatures in conjunction with the recycling an excellent balance between purity, yield and selectivity could be achieved, while simultaneous allowing for a considerable reduction in waste.

Moreover, this approach can efficiently be transferred to the industrial large-scale preparation of 1-alkenes and therefore the corresponding 1,2-alkanediols, especially from an ecological and economical point of view.

**Example 6:** Preparation of bio-alkenes based on the continuous addition of 1-alcohol and distillation bottoms - Recycling process according to the invention:

Bio-alkenes based on the dehydration of 1-alcohol according to Example 6 were prepared as indicated above and based on the continuous addition of 1-alcohol and distillation bottoms (recycling process according to the invention) and the following reaction conditions:

| | |
|---|---|
| Distillation Bottoms | 42.8 % 1-heptanol, 55.5 % diheptyl ether and 1.7 % of unknown residues |
| Dosage | 0.5 ml/min |
| 1-Heptanol | 2.0 ml/min |
| $N_2$ | 8 $ml_n$/min |

| | |
|---|---|
| Fixed-bed | 14 mm ID x 500 mm (77.0 ml) |
| Catalyst | 63.0 g Alumina spheres (1.0 mm) from Sasol |

The catalyst bed was preheated and as soon as the desired temperature was reached, fresh 1-heptanol is pumped at a rate of 2.0 ml/min and distillation bottoms comprising diheptyl ether and 1-heptanol were pumped at a rate of 0.5 ml/min on top of the evaporation zone (corresponding to approximately 20 % recirculation, i.e., a volume partition of 20% of the bottoms/recycling compounds relative to the total dosage). After one hour a steady state was reached with a temperature of 276 °C inside the catalyst bed. Subsequently, within 48 hours, 4717.4 g of 1-heptanol and 1164.7 g of distillation bottoms were dosed, and 5812.0 g of the as-obtained product collected. From the product 635.0 g of a water layer were separated and the remaining 5177.0 g of organic phase were purified by distillation at 450 mmbar using a short 30 cm packed column equipped with a column head.

In summary, the mass reaction balance was as follows:

| | |
|---|---|
| Water: | 635.0 g |
| 1-Heptene, pure: | 2925.7 g |
| 1-Heptene, for re-distillation: | 244.9 g (middle run fraction) |
| Distillation bottoms: | 1982.7 g |
| Loss: | 23.7 g |
| $\Sigma$ g | 5812.0 g |

Table 7 shows the corresponding composition of the as-obtained product after removal of water, the isolated 1-heptene product, the 1-heptene obtained in a middle distillation run fraction which is to be re-distilled (to avoid waste and product losses) and finally the composition of the remaining distillation bottoms after removal of the 1-heptene.

**Table 7:** Corresponding product composition by weight.

| Compound [wt.-%] | Product as-obtained | Isolated 1-Heptene | 1-Heptene for re-distillation (middle run fraction) | Remaining bottoms for recirculation |
|---|---|---|---|---|
| 1-Heptene | 59.2 | 96.4 | 89.6 | 0 |
| Heptene Isomers | 2.1 | 3.1 | 4.8 | 0 |
| 1-Heptanol | 14.6 | 0.1 | 1.9 | 37.6 |
| Diheptyl Ether | 23.2 | 0.2 | 0.2 | 60.1 |
| Unknown | 0.9 | 0.2 | 3.3 | 2.2 |
| Compound [g] | | | | |
| 1-Heptene | 3066.0 | 2819.7 | 219.4 | 0 |
| Heptene Isomers | 106.6 | 89.4 | 11.8 | 0 |
| 1-Heptanol | 754.5 | 3.1 | 4.7 | 746.2 |
| Diheptyl Ether | 1201.6 | 7.3 | 0.6 | 1192.1 |
| Unknown | 47.9 | 6.1 | 8.2 | 43.4 |
| Σ [g] | 5177.0 | 2925.7 | 244.9 | 1982.7 |
| Conversion [%] | 87.0 | - | - | - |
| Selectivity [%] | 71.7 | - | - | - |
| Yield [%] | 62.4 | - | - | - |
| Purity [%] | 96.6 | 96.9 | 94.9 | - |

A GC measurement of the educts shows a conversion of 9.3 % due to the diheptyl ether content contained in the distillation bottoms. In total 5375.0 g of 1-heptanol and 507.1 g of diheptyl ether are dosed corresponding to a diheptyl ether content of 8.6 %.

A GC analysis of the products indicates a 1-heptanol conversion of 87.0 %, a 1-heptene selectivity of 71.7 % and a 1-heptene yield of 62.4 %. Losses can be mainly attributed to unreacted diheptyl ether and to a less pronounced formation of heptene isomers.

Surprisingly, a simple distillation is resulting in 2925.7 g of a 1-heptene fraction having an excellent purity of 96.4 %. Referring to the total feed this corresponds to an isolated 1-heptene yield of 56.3 % (28.724 mol). In addition, a small (244.9 g) middle run fraction contained further 4.4 % of crude 1-heptene yield (2.234 mol) for further isolation via re-distillation.

The 1982.7 g distillation bottoms contain mainly unwanted diheptyl ether by-product and to less extend unconverted 1-heptanol. This means 21.8 % of diheptyl ether (5.560 mol) crude yield available for recycling is obtained. However, 635.0 g (35.248 mol) of the water formed correspond to a yield of 72.5 %.

**Example 7:** Preparation of bio-alkenes based on the continuous addition of 1-alcohol and distillation bottoms - Recycling process according to the invention:

Bio-alkenes based on the dehydration of 1-alcohol according to Example 7 were prepared as indicated above and based on the continuous addition of 1-alcohol and distillation bottoms (recycling process according to the invention) and the following reaction conditions:

| | |
|---|---|
| Distillation Bottoms | 44.2 % 1-heptanol, 55.1 % diheptyl Ether and 0.7 % of unknown residues |
| Dosage | 1.0 ml/min |
| 1-Heptanol | 1.5 ml/min |
| $N_2$ | 8 $ml_n$/min |

(continued)

| | |
|---|---|
| Fixed-bed | 14 mm ID x 500 mm (77.0 ml) |
| Catalyst | 61.5 g Alumina spheres (1.0 mm) from Sasol |

The catalyst bed was preheated and as soon as the desired temperature was reached, fresh 1-heptanol was pumped at a rate of 1.5 ml/min and distillation bottoms comprising diheptyl ether and 1-heptanol were pumped at rate of 1.0 ml/min on top of the evaporation zone (corresponding to approximately 40 % recirculation, i.e. a volume partition of 40 % of the bottoms relative to the total dosage). After one hour a steady state was reached with a temperature of 276 °C inside the catalyst bed. Subsequently, within 30 hours 2211.3 g of 1-heptanol and 1456.2 g of distillation bottoms were dosed, and 3589.0 g of the product collected. From the as-obtained product 360.3 g of water were separated and the remaining 3228.7 g of organic phase purified by distillation at 450 mbar using a short 30 cm packed column equipped with a column head.

In summary, the mass reaction balance was as follows:

| | |
|---|---|
| Water: | 360.3 g |
| 1-Heptene, pure: | 1944.0 g |
| 1-Heptene, for re-distillation: | 244.9 g (optional middle run fraction) |
| Distillation bottoms: | 1269.0 g |
| Loss: | 15.7 g |
| Σ g | 3589.0 g |

Table 8 shows the corresponding product composition of the as-obtained product after removal of water, the isolated 1-heptene product and the remaining distillation bottoms after removal of the 1-heptene.

**Table 8:** Corresponding product composition by weight.

| Compound [wt.-%] | Product as-obtained | Isolated 1-Heptene | Bottoms for recirculation |
|---|---|---|---|
| 1-Heptene | 59.0 | 96.1 | 1.0 |
| Heptene Isomers | 1.9 | 3.2 | 0.1 |
| 1-Heptanol | 15.2 | 0.4 | 38.0 |
| Diheptyl Ether | 22.9 | 0.0 | 58.8 |
| Unknown | 1.0 | 0.2 | 2.0 |
| Compound [g] | | | |
| 1-Heptene | 1906.2 | 1868.8 | 13.2 |
| Heptene Isomers | 61.7 | 62.7 | 0.8 |
| 1-Heptanol | 489.7 | 7.8 | 482.3 |
| Diheptyl Ether | 739.6 | 0.5 | 746.8 |
| Unknown | 31.5 | 4.2 | 25.8 |
| Σ [g] | 3228.7 | 1944.0 | 1269.0 |
| Conversion [%] | 86.5 | - | - |
| Selectivity [%] | 72.1 | - | - |
| Yield [%] | 62.3 | - | - |
| Purity [%] | 96.9 | 96.8 | 94.7 |

A GC analysis of the educt composition indicates a conversion of 23.4 % due to the high diheptyl ether content within the distillation buttons. In total 2859.5 g 1-heptanol and 808.0 g diheptyl ether are dosed, corresponding to a diheptyl ether content of 22.0 %.

GC product analysis indicates a 1-heptanol conversion of 86.5 %, a 1-heptene selectivity of 72.1 % and a 1-heptene yield of 62.3 %. Losses can be mainly attributed to unreacted diheptyl ether and to less a less pronounced

formation of heptene isomers.

Surprisingly, simple distillation is resulting in 1944.0 g of a 1-heptene fraction having an excellent purity of 96.4 %. Referring to the total feed this corresponds to an isolated 1-heptene yield of 59.4 % (19.086 mol).

The 1269.0 g distillation bottoms contain mainly the unwanted diheptyl ether by-product and to less extend unconverted 1-heptanol. This means that 21.7 % of a diheptyl ether (3.483 mol) crude yield were obtained. This example clearly demonstrates that under suitable conditions the product contains less diheptyl ether than the educt stream. Furthermore, conditions are well chosen and close to an optimal operating point at which a flow equilibrium is achieved between diheptyl ether formation and recirculation (i.e. an almost perfect flow equilibrium is achieved at a recirculation rate of approximately 40 %, i.e. a volume partition of 40 % of the bottoms relative to the total dosage). However, 360.3 g (20.000 mol) of water were formed corresponding to a yield of 70.5 %.

These conditions are suitable for mass production.

**Example 8:** Acid value and induction period of composition comprising at least two different alkanediols according to the invention having the same chain length - Oxipres test to simulate product protection (homogeneous chain lengths of the alkanediols):

Surprisingly, it was found, as described in more detail in the following example, that oxidative degradation of a composition according to the present invention can considerably be reduced or minimized with an antioxidant. This means that the composition is more stable against oxidative degradation. This comes along with a lower acid value, which is a measure of decomposition of triglycerides, and a less rancid odour. By contrast, 1,2-alkanediols alone do not show any antioxidant performance.

An Oxipres test was performed in order to evaluate the antioxidative capacity of different test samples as described below.

Test samples:

| | |
|---|---|
| Sample A1: | Sunflower oil without additives |
| Sample B1: | Sunflower oil plus 0.1 % tocopherol |
| Sample C1: | Sunflower oil plus 0.1 % tocopherol plus 0.5 % 1,2-hexanediol |
| Sample D1: | Sunflower oil plus 0.1 % tocopherol plus 0.5 % 2,3-hexanediol |
| Sample E1: | Sunflower oil plus 0.1 % tocopherol plus 0.5 % of an alkanediol blend of 95 % 1,2-hexanediol and 5 % 2,3-hexanediol |

Test procedure: For the evaluation of the antioxidative capacity of the above-described samples, an Oxipres test was conducted. The Oxipres method is bases on oxygen consumption at high temperatures and pressures and allows the determination of oxidative resistance (shelf life) of e.g. oils. The test runs under elevated pressure and temperature, where the process is accelerated. In the Oxipres test the sample is placed inside a hermetically closed iron vessel that is subjected to high oxygen pressures and temperatures of 90 °C to 120 °C. The above-described samples were treated in the Oxipres device for 48 hours at 80 °C and 5 bar pressure and of the induction period determined. The consumption of oxygen results in a pressure drop in the vessel during the test. A higher decrease of pressure indicates more consumption of oxygen and a higher oxidation of the test product. Oils with a high degree of unsaturation are most susceptible to autoxidation.

The induction period is the period during which a fat or oil shows stability against oxidation because of its content of antioxidants, either naturally or added. In the test, the antioxidants are oxidized preferentially before the oxidizable fat or oil is oxidized. Thus, the antioxidants protect the fat or oil against oxidation. After this there is a sudden and large consumption of oxygen, and the fat becomes rancid. This can be defined by the induction period (IP) in hours. A lower IP correlates with a faster oxidation.

Equipment: The tests were performed with an Oxipres device ex Mikrolab. The instrument is a modification of the bomb method (ASTM D941), which is based on oxidation with oxygen.

Before and after Oxipres treatment additionally an odour evaluation of the samples was performed and the acid value of the samples before and after Oxipres treatment was determined.

Principle of acid value determination: The acid value is determined based on the neutralization of the free acids by titration with the ethanolic or aqueous solution of potassium hydroxide. It shows the number of milligram (mg) KOH required in order to neutralize the free acids in 1 g test substance. The titration according to the IFU respectively § 64 LFGB (formerly § 35 LMBG) is carried out potentiometrically with potassium hydroxide solution to a pH-value of 8.1.

**Table 9:** Results: Induction period (IP) and acid value after Oxipres treatment for hexanediol isomers (C6).

| Sample | Induction period [h] | Acid value [mg KOH/g] after treatment |
|---|---|---|
| A1 | 24.5 | 8.7 |
| B1 | 31.2 | 5.7 |
| C1 | 30.3 | 6.6 |
| D1 | 31.6 | 5.0 |
| E1 | 31.2 | 4.5 |

The above results clearly show that Sample E1 (comprising a blend of 95 % 1,2-hexanediol and 5% 2,3-hexanediol) shows the lowest acid value among the samples and the longest induction period. The higher the acid number, the lower the quality of the composition, meaning that as the acid number increases, the oxidation stability decreases. Sample E1 shows a prolonged induction period indicating a longer shelf life. This means that the samples are more stable against oxidative degradation than the comparative samples (see Table 9).

The above advantageous results are confirmed by a lower acid value. The acid value is defined as the number of milligrams of potassium hydroxide required to neutralize the free fatty acids present in one gram of fat. It is a relative measure of rancidity as free fatty acids are normally formed during decomposition of triglycerides. Hence, the acid value correlates with the rancidity degree in an oil. The acid value of Sample E1 is 4.5 mg KOH/g after the treatment, while the single isomeric hexanediols (Samples C1 and D1) show acid values of about 6.6 and 5.0, respectively, indicating a lower efficiency in view of oxidation stability for compositions or formulations comprising only one isomeric form of hexanediol while a combination of a 1,2-alkanediol with a 2,3-alkanediol shows a synergistic effect.

As it is demonstrated by the above example, the induction period of the samples can be extended with an antioxidant (e.g. tocopherol) and can be even more, i.e., synergistically, improved by combing 1,2-alkanediols with 2,3-alkanediols in a 95 : 5 ratio or a 99 : 1 ratio, respectively. Hence, it is proven that the oxidation process of sample including an oil can be decelerated with the concurrent use of an antioxidant and a specific combination of alkanediols.

Comparable improved results could also be achieved for a combination of 1,2-heptanediol and 2,3-heptanediol in a 95 : 5 ratio and a 99 : 1 ratio, respectively, indicating that already small amounts of the 2,3-homologues are sufficient for an improved synergistic effect.

Again, an Oxipres test was performed in order to evaluate the antioxidative capacity of different test samples as described below (see also Table 10).

Test samples:

| | |
|---|---|
| Sample A2: | Sunflower oil without additives |
| Sample B2: | Sunflower oil plus 0.1 % tocopherol |
| Sample C2: | Sunflower oil plus 0.1 % tocopherol plus 0.5 % 1,2-heptanediol |
| Sample D2: | Sunflower oil plus 0.1 % tocopherol plus 0.5 % 2,3-heptanediol |
| Sample E2: | Sunflower oil plus 0.1 % tocopherol plus 0.5 % of an alkanediol blend of 95 % 1,2-heptanediol and 5 % 2,3-heptanediol |
| Sample F2: | Sunflower oil plus 0.1 % tocopherol plus 0.5 % of an alkanediol blend of 99 % 1,2-heptanediol and 1 % 2,3-heptanediol |
| Sample G2: | Sunflower oil plus 0.1 % tocopherol plus 0.5 % of an alkanediol blend of 98 % 1,2-heptanediol and 2 % 2,3-heptanediol |

**Table 10:** Results: Induction period (IP) and acid value after Oxipres treatment for heptanediol isomers (C7).

| Sample | Induction period [h] | Acid value [mg KOH/g] after treatment |
|---|---|---|
| A2 | 22.6 | 8.5 |
| B2 | 29.5 | 6.1 |
| C2 | 31.2 | 5.1 |

(continued)

| Sample | Induction period [h] | Acid value [mg KOH/g] after treatment |
|---|---|---|
| D2 | 32.0 | 6.1 |
| E2 | 31.7 | 5.2 |
| F2 | 32.0 | - |
| G2 | 32.0 | - |

Sensory evaluation: In this context a sensory evaluation further revealed that Sample E2 (sunflower oil plus 0.1 % tocopherol plus 0.5 % of an alkanediol blend of 95 % 1,2-heptanediol and 5 % 2,3-heptanediol) has a better odour, i.e., the sample showed a less rancid odour than the comparative examples and e.g. Sample C2 (sunflower oil plus 0.1 % tocopherol plus 0.5 % 1,2-heptanediol) without additional 2,3-heptanediol, confirming a synergistic effect between the two isomeric alkanediol components. As it is demonstrated by the above test, oxidative degradation can be reduced or minimized with an antioxidant (tocopherol) and can be even more improved, i.e., boosted, with the addition of and antioxidant in combination with a mixture of two isomeric alkanediols, e.g. in a ratio of 95 : 5.

Improved induction periods as well as improved acid numbers compared to samples not comprising an antioxidant and/or alkanediol mixture could also be achieved for a combination of 1,2-octanediol and 2,3-otanediol (C8-alkanediol) in a 95 : 5 ratio (Samples A3, B3 and C3), for a combination of 1,2-nonanediol and 2,3-nonanediol (C9-alkanediol) in a 95 : 5 ratio (Samples A4, B4 and C4), as well as for a combination of 1,2-decanediol and 2,3-decanediol (C10-alkanediol) in a 95 : 5 ratio (Samples A5, B5, C5, D5 and E5).

Test samples:

Sample A3: Sunflower oil without additives
Sample B3: Sunflower oil plus 0.1 % tocopherol
Sample C3: Sunflower oil plus 0.1 % tocopherol plus 0.5 % of an alkanediol blend of 95 % 1,2-octanediol and 5 % 2,3-octanediol
Sample A4: Sunflower oil without additives
Sample B4: Sunflower oil plus 0.1 % tocopherol
Sample C4: Sunflower oil plus 0.1% tocopherol plus 0.5 % of an alkanediol blend of 95 % 1,2-nonanediol and 5 % 2,3-nonanediol
Sample A5: Sunflower oil without additives
Sample B5: Sunflower oil plus 0.1% tocopherol
Sample C5: Sunflower oil plus 0.1% tocopherol plus 0.5 % 1,2-decanediol
Sample D5: Sunflower oil plus 0.1% tocopherol plus 0.5 % 2,3-decanediol
Sample E6: Sunflower oil plus 0.1 % tocopherol plus 0.5 % of an alkanediol blend of 95 % 1,2-decanediol and 5 % 2,3-decanediol
Sample F6: Sunflower oil plus 0.1 % tocopherol plus 0.5 % of an alkanediol blend of 50 % 1,2-decanediol and 50 % 2,3-decanediol

The corresponding results are shown in Table 11 below.

**Table 11:** Results: Induction period (IP) and acid value after Oxipres treatment for further alkanediol isomers.

| Sample | Induction period [h] | Acid value [mg KOH/g] after treatment |
|---|---|---|
| A3 | 24.5 | 8.7 |
| B3 | 31.2 | 5.7 |
| C3 | 30.8 | 6.8 |
| A4 | 16.2 | 13.7 |
| B4 | 23.5 | 10.4 |

(continued)

| Sample | Induction period [h] | Acid value [mg KOH/g] after treatment |
|---|---|---|
| C4 | 22.5 | 9.6 |
| A5 | 24.5 | 8.7 |
| B5 | 31.2 | 5.7 |
| C5 | 31.7 | 5.5 |
| D5 | 31.8 | 5.4 |
| E5 | 31.9 | 5.3 |
| F5 | - | 5.6 |

Analogous experiments using Symdecanox HA (caprylic/capric triglyceride, hydroxy methoxyphenyl decanone) instead of tocopherol likewise show an improved oxidation stability of the samples comprising a combination of at least two different alkanediols in a 95 : 5 ratio (Samples A6 to E6 and A7 to E7; see Table 12).

Test samples:

| | |
|---|---|
| Sample A6: | Sunflower oil without additives |
| Sample B6: | Sunflower oil plus 0.1 % Symdecanox HA |
| Sample C6: | Sunflower oil plus 0.1 % Symdecanox HA plus 0.5 % 1,2-heptanediol |
| Sample D6: | Sunflower oil plus 0.1 % Symdecanox HA plus 0.5 % 2,3-heptanediol |
| Sample E6: | Sunflower oil plus 0.1 % Symdecanox HA plus 0.5 % of an alkanediol blend of 95 % 1,2-heptanediol and 0.5 % 2,3-heptanediol |
| Sample A7: | Sunflower oil without additives |
| Sample B7: | Sunflower oil plus 0.1 % Symdecanox HA |
| Sample C7: | Sunflower oil plus 0.1 % Symdecanox HA plus 0.5 % 1,2-octanediol |
| Sample D7: | Sunflower oil plus 0.1 % Symdecanox HA plus 0.5 % 2,3-octanediol |
| Sample E7: | Sunflower oil plus 0.1% Symdecanox HA plus 0.5 % of an alkanediol blend of 95 % 1,2-octanediol and 5 % 2,3-octanediol |

**Table 12:** Results of induction period (IP) and acid value after Oxipres treatment for further alkanediol isomers using in combination with Symdecanox HA.

| Sample | Induction period [h] | Sample | Induction period [h] |
|---|---|---|---|
| A6 | 23.1 | A7 | 23.1 |
| B6 | 24.4 | B7 | 24.4 |
| C6 | 24.4 | C7 | 23.9 |
| D6 | 24.7 | D7 | 24.0 |
| E6 | 25.3 | E7 | 25.1 |

Based on the above results, it can be concluded that the synergistic effect is not dependent on the antioxidant used. Additionally, minor amounts of the 2,3-alkanediol in combination of with the corresponding 1,2-alkanediols are sufficient to achieve a pronounced synergistic effect.

Good results were also achieved when using Pentaerythrityl Tetra-di-t-butyl Hydroxyhydrocinnamate (Tinogard TT) instead of Symdecanox HA.

**Example 9:** Acid value and induction period of composition comprising at least two different alkanediols with different chain lengths according to the invention - Oxipres test to simulate product protection (heterogeneous chain lengths of the alkanediols):

Furthermore, it was surprisingly found, as described in more detail in the following example, that the oxidative degradation can considerably be reduced or minimized by using a combination of at least two different alkanediols, i.e. 1,2-alkanediols with 2,3-alkanediols having different chain lengths, in a 95 : 5 ratio, whereby the effect is more pronounced in a synergistical manner compared to the addition of one single 1,2-alkanediol or 2,3-alkanediol. This means that the composition comprising the mixtures according to the invention is more stable against oxidative degradation as shown in Table 13 (Samples A1 to E1: 1,2-C6 and 2,3-C7; Samples A2 to E2: 1,2-C7 and 2,3-C8).

The Oxipres test was conducted as described in Example 8.

Test samples:

| | |
|---|---|
| Sample A1: | Sunflower oil without additives |
| Sample B1: | Sunflower oil plus 0.1 % tocopherol |
| Sample C1: | Sunflower oil plus 0.1 % tocopherol plus 0.5 % 1,2-heptanediol |
| Sample D1: | Sunflower oil plus 0.1 % tocopherol plus 0.5 % 2,3-hexanediol |
| Sample E1: | Sunflower oil plus 0.1 % tocopherol plus 0.5 % of an alkanediol blend of 95 % 1 ,2-heptanediol and 5 % 2,3-hexanediol |
| Sample A2: | Sunflower oil without additives |
| Sample B2: | Sunflower oil plus 0.1 % tocopherol |
| Sample C2: | Sunflower oil plus 0.1 % tocopherol plus 0.5 % 1,2-heptanediol |
| Sample D2: | Sunflower oil plus 0.1 % tocopherol plus 0.5 % 2,3-octanediol |
| Sample E2: | Sunflower oil plus 0.1 % tocopherol plus 0.5 % of an alkanediol blend of 95 % 1 ,2-heptanediol and 5 % 2,3-octanediol |

**Table 13:** Results: Induction period (IP) and acid value after Oxipres treatment for hetero combinations of alkanediol isomers.

| Sample | Induction period [h] | Sample | Induction period [h] |
|---|---|---|---|
| A1 | 25.9 | A2 | 25.9 |
| B1 | 31.2 | B2 | 31.2 |
| C1 | 32.3 | C2 | 32.3 |
| D1 | 32.9 | D2 | 31.2 |
| E1 | 33.2 | E2 | 32.2 |

Based on the above results it can be concluded that a combination of 1,2-alkanediols with 2,3-alkanediols having different chain lengths allows for the preparation of more stable compositions.

The examples show that a combination of different isomeric forms of alkanediols either having the same chain length or, alternatively, a different chain length, allows for the preparation of compositions that are more stable against oxidative degradation and thus have a longer shelf life based on a synergistic effect based on a synergistic effect of the alkanediol combinations.

Additionally, it was experimentally found that samples, in particular samples comprising a mixture of different isomeric alkanediols having different chain lengths show improved performances and stabilities against oxidative degradation if the relative ratio of the 1,2-alkanediol to the 2,3-alkanediol is in the range of 95 : 5 to 99.9 : 0.1 (see Table 14).

Test samples:

Sample E2.1: Sunflower oil plus 0.1% tocopherol plus 0.5 % of an alkanediol blend of 95 % 1 ,2-heptanediol and 5 % 2,3-hexanediol

Sample E2.2: Sunflower oil plus 0.1 % tocopherol plus 0.5 % of an alkanediol blend of 50 % 1,2-heptanediol and 50 % 2,3-hexanediol

Sample E3.1: Sunflower oil plus 0.1 % tocopherol plus 0.5 % of an alkanediol blend of 95 % 1 ,2-heptanediol and 5 % 2,3-octanediol

Sample E3.2: Sunflower oil plus 0.1 % tocopherol plus 0.5 % of an alkanediol blend of 50 % 1 ,2-heptanediol and 50 % 2,3-octanediol

**Table 14:** Results: Induction period (IP) and acid value after Oxipres treatment for heterogeneous combinations of alkanediol isomers in dependence of their relative ratio.

| Sample | Induction period [h] | Acid value [mg KOH/g] after treatment |
|---|---|---|
| E2.1 (95 : 5) | 33.2 | 3.9 |
| E2.2 (50 : 50) | 33.0 | 4.1 |
| E3.1 (95 : 5) | 32.2 | 5.2 |
| E3.2 (50 : 50) | 31.7 | 5.3 |

**Example 10:** Antioxidant potential on ex vivo skin biopsies (in aqueous/alcoholic (ethanolic) test samples):

In a biological context, reactive oxygen species (ROS) are formed as a natural by-product of the normal metabolism of oxygen and have important roles in cell signalling and homeostasis. However, at times of environmental stress (for example upon UV or heat exposure), ROS levels can increase dramatically. Surprisingly, it turns out that the composition according to the present invention has a significant antioxidative capacity and a superior ROS scavenging activity, i.e., a better ROS score.

A dichlorofluorescein test (DCF) assay was performed in order to determine the amount of reactive oxygen species (ROS) on ex vivo skin treated with different test samples in a lipophilic or an aqueous/alcoholic system as described below.

DCF assay - assay principle: Ex vivo skin was incubated with 2',7'-dichlorodihydrofluorescein diacetate at 37 °C, 5 % $CO_2$. After PBS washing, the samples were exposed or not to cumene hydroperoxide. Immediately after exposure, ex vivo skin samples were frozen in liquid nitrogen and 5 μm cryostat sections were made and fixed with acetone to allow the visualization of fluorescence generated by ROS in cells of the reconstructed skins. The resulting fluorescence was measured at EX/EM 504/524 nm. Green fluorescence was quantified in ex vivo skin using ImageJ software. The depth of immunostaining was measured as follows: green DCFH-DE positive cells were automatically detected using Histolab software and the distance between dermal epidermal junction and the deepest positive cells were measured in each condition. Means were compared using a student's test. Two means were considered statistically different when $p < 0.005$.

The image acquisition was performed by using Olympus BX51 microscope and Olympus DP70 camera. For each skin sample two skin sections have been taken and the related fluorescent images acquired and analysed. Thus, for each test condition, 12 images have been acquired and analysed (i.e. 12 data). The analysis of fluorescence has been performed within the dermis area. For each image the upper dermis has been analysed by evaluating the fluorescence through modified Image-J application (NIH, USA). The analysed area is selected from the upper part by following the perimeter of the basal lamina, to the deep dermis, by carefully avoiding the risk of including irregularities and agglomerates, such as blood vessels, sebaceous glands, hair follicles. The obtained value has been normalized upon the dimension of the selected area.

In addition, as positive control an AOX mix is always co-tested, i.e., as benchmark for the ROS analysis. The AOX mix is a mixture of the following antioxidants: 15 % vitamin C, 1 % vitamin E, 0.5 % ferulic acid in a EtOH/$H_2O$ (50 : 50) mixture.

ROS score method description: The pigmentation score is based on the following process steps:

(1) Analysis of the image based on pixel grey intensities;
(2) Selection of an informative area excluding the pixel area not covered from tissue;

(3) Transformation of pixel grey intensities in degrees of L*;
(4) Normalization of the obtained values on the ratio between the selected area and the area of the slide.

An increased level of ROS (reactive oxygen species) led to an increased amount of fluorescence.

DCF-Assay in biopsy antioxidation test: 1,2-alkanediols or 2,3-alkanediols for aqueous/alcoholic test samples:

The dichlorofluorescein test (DCF) as described before was performed with different test samples in an aqueous/alcoholic (ethanolic) test system as described below:

Test samples:

1) Vehicle: EtOH/$H_2O$ in 6 : 4 ratio by weight without additives
2) Vehicle plus 50 ppm Dihydroavenanthramide D (DHAvD)
3) Vehicle plus 50 ppm Dihydroavenanthramide D (DHAvD) plus 0.5 % 1,2-heptanediol
4) Vehicle plus 50 ppm Dihydroavenanthramide D (DHAvD) plus 0.5 % 2,3-heptanediol
5) Vehicle plus 50 ppm Dihydroavenanthramide D (DHAvD) plus alkanediol blend (95 % 1,2-heptanediol and 5 % 2,3-heptanediol)

The ROS scores of the above specified samples are summarized in Table 15 and Figure 3.

The results clearly demonstrate that the addition of 1,2-alkanediols or 2,3-alkanediols lead to an improvement of the ROS scores. Interestingly, the combination of 5 % 2,3-alkanediols with 95 % of 1,2-alkanediols shows efficiencies comparable to those of 100 % 2,3-alkanediols, proving a synergistic effect between the 1,2- and 2,3-alkanediols, even if only minor portions of the 2,3-alkanediol are added. From the above it can be concluded that the addition of small portions of an 2,3-alkanediol to an 1,2-alkanediol results in considerably reduced ROS scores of said 1,2-alkanediol in a synergistic manner, thus showing an improved ROS scavenging efficacy (C7 ≙ heptanediol).

**Table 15:** Results: ROS score (C7: heptanediol).

| Test | No cumene | Cumene hydroperoxide 0.5 M | | | | | | |
|---|---|---|---|---|---|---|---|---|
| | untreated | untreated | AOX mix | ETOH/ $H_2O$ (6: 4) | 50 ppm DHAvD | 50 ppm DHAvD + 0.5 % 1,2-C7 | 50 ppm DHAvD + 0.5 % 2,3-C7 | 50 ppm DHAvD + 0.5 % (1,2-C7 + 2,3-C7 (95 : 5)) |
| 1 | - | 2.397 | - | 0.894 | 1.552 | 0.748 | 0.116 | 0.694 |
| 2 | 0.132 | 3.447 | 0.021 | 3.201 | 2.083 | 0.709 | 0.414 | 0.322 |
| 3 | 0.120 | 2.359 | 0.305 | 2.012 | 1.367 | 0.288 | 0.919 | 0.289 |
| 4 | 0.163 | 2.648 | 0.067 | 1.044 | 2.338 | 0.874 | 0.552 | 0.802 |
| 5 | 0.061 | 4.630 | 0.167 | 2.820 | 1.245 | 0.071 | 0.274 | 0.409 |
| 6 | 0.110 | 0.680 | 0.263 | 3.397 | 1.929 | 0.079 | 1.685 | 0.230 |
| 7 | 0.008 | 2.754 | 0.227 | 2.535 | - | 1.060 | 1.009 | - |
| 8 | 0.186 | 1.355 | 0.330 | 0.931 | 1.230 | 0.718 | 0.230 | 0.921 |
| 9 | 0.088 | 0.994 | 0.212 | 0.828 | 2.420 | 1.073 | 0.344 | 1.693 |
| 10 | 0.001 | 2.204 | 0.217 | 3.946 | 1.602 | 1.243 | 0.522 | - |
| 11 | 0.159 | 1.161 | 0.314 | 1.230 | 1.706 | 1.580 | 0.414 | 0.661 |
| 12 | 0.057 | 1.431 | 0.021 | 1.316 | 1.466 | 1.375 | 1.357 | 0.481 |
| **Mean Score** | **0.10** | **2.17** | **0.19** | **2.01** | **1.72** | **0.82** | **0.65** | **0.65** |

(continued)

| Test | No cumene | Cumene hydroperoxide 0.5 M | | | | | | |
|---|---|---|---|---|---|---|---|---|
| | untreated | untreated | AOX mix | ETOH/ $H_2O$ (6: 4) | 50 ppm DHAvD | 50 ppm DHAvD + 0.5 % 1,2-C7 | 50 ppm DHAvD + 0.5 % 2,3-C7 | 50 ppm DHAvD + 0.5 % (1,2-C7 + 2,3-C7 (95 : 5)) |
| St. Dev | 0.06 | 1.14 | 0.11 | 1.12 | 0.42 | 0.49 | 0.49 | 0.43 |
| SEM | 0.02 | 0.33 | 0.03 | 0.32 | 0.13 | 0.14 | 0.14 | 0.14 |
| D percent vs. untreated / No cumene | | 2101 % | 97 % | 1940 % | 1645 % | 729 % | 562 % | 559 % |
| D percent vs. untreated cumene | | | - 91 % | - 7 % | - 21 % | - 62 % | - 70 % | - 70 % |
| D percent vs. - A cumene | | | | | -14% | - 59 % | - 68 % | - 68 % |

**Example 11:** Antimicrobial activity: Minimum Inhibitory Concentration (MIC) - Synergistic activity:

The Minimum Inhibitory Concentration (MIC) test is a test on growth inhibition. The estimation of MICs is executed in 96 well plates. Through the comparison of bacterial growth with positive and negative controls via optical density (OD), different concentrations of given test substances are evaluated.

Bacteria were cultivated under adjusted conditions based on information of the German Collection of Microorganisms and Cell Cultures (DSMZ) and stored in 50 % glycerol prior to use. Afterwards, the following method was applied: Growth medium (according to microorganism) was added to each well of the microplate. Then, the test-substances were added in different concentrations, positive controls (water controls only with medium) and negative controls (benchmarks according to organism). Finally, the microorganisms were added in a concentration of 106 CFU / ml (CFU = colony forming units). The incubation of the microplates was carried out at appropriate conditions.

According to the resulting growth, substance concentrations are labelled either as inhibiting or not and MIC is defined as the lowest concentration where a complete growth inhibition is visible. Experiments at concentration limits (highest concentration labelled as growth and lowest concentration labelled as inhibiting) are performed at least twice.

Minimum Inhibitory Concentration (MIC) are given in ppm. "> 5000" indicates that 5000 ppm was tested, and no growth inhibition found, higher values were not investigated.

The synergistic activity of different combinations of 1,2-nonanediol (C9 ≙ nonanediol) and 1,2-heptanediol (C7 ≙ heptanediol) as components A and C in combination with different antimicrobial compounds (component B) was determined by measuring the corresponding Minimum Inhibitory Concentrations (MIC).

To verify that the tested combinations act synergistically against microorganisms of the natural microbiota of mammalian skin, a corresponding experiment was conducted with *Staphylococcus aureus,* a well-known pathogen on the skin. The synergistic activities of 1,2-nonanediol (1,2-C9), 1,2-heptanediol (1,2-C7), 2,3-nonanediol (2,3-C9), 2,3-heptanediol (2,3-C7) and different antimicrobial compounds were determined by measuring the MIC of the mixture as indicated above.

In order to calculate the synergy index with MIC values, the Kull equation (equation 6) was used as follows:

$$SI = (MIC_{mix} \times P_A) / MIC_A + (MIC_{mix} \times P_B) / MIC_B + (MIC_{mix} \times P_C) / MIC_C$$

*Equation (6): Minimum Inhibitory Concentration*

wherein

| | |
|---|---|
| SI | is the Synergy Index according to Kull |
| $MIC_A$ | is the MIC value for Substance A in ppm |
| $MIC_B$ | is the MIC value for Substance B in ppm |
| MICc | is the MIC value for Substance C in ppm |
| $MIC_{mix}$ | is the MIC value for the mixture of substances A and B and C in ppm |

$P_A$ is the proportion of the substance A in the mixture
$P_B$ is the proportion of the substance B in the mixture
$P_C$ is the proportion of the substance C in the mixture

**Table 16:** MIC values and synergy index of compositions according to the present invention with different antimicrobials.

| A | B | C | Portion A | Portion B | Portion C | $MIC_A$ | $MIC_B$ | $MIC_C$ | $MIC_{mix}$ | SI |
|---|---|---|---|---|---|---|---|---|---|---|
| 1,2-C7 | Benzyl alcohol | 2,3-C7 | 0.5 | 0.45 | 0.05 | 7500 | 5000 | 20000 | 5000 | 0.80 |
| 1,2-C7 | o-Cymen-5-ol | 2,3-C7 | 0.5 | 0.45 | 0.05 | 7500 | 250 | 20000 | 500 | 0.93 |
| 1,2-C9 | Capryl hydroxamic acid | 2,3-C9 | 0.5 | 0.45 | 0.05 | 1000 | 1000 | 2500 | 500 | 0.49 |
| 1,2-C9 | Benzyl alcohol | 2,3-C9 | 0.5 | 0.45 | 0.05 | 1000 | 5000 | 2500 | 1000 | 0.61 |
| 1,2-C9 | o-Cymen-5-ol | 2,3-C9 | 0.5 | 0.45 | 0.05 | 1000 | 250 | 2500 | 250 | 0.58 |

* Nonanediol = C9
Heptanediol = C7

For all samples synergistic effects regarding the antimicrobial efficacy were found with SI values below 1. This means that with the addition of a composition according to the present invention comprising a mixture of 1,2-alkanediols and 2,3-alkanediols, the concentration of the antimicrobial component can be efficiently reduced without negatively affecting the antimicrobial effect. Hence, the use of a composition according to the present invention comprising minor amounts of the 2,3-component (e.g. ratio of 90 : 10) allows for the use of less antimicrobial components in a cosmetic or pharmaceutical composition for achieving the same antimicrobial effect based on a synergistic effect, compared to a cosmetic or pharmaceutical composition comprising the same antimicrobial component but without the use of said compositions.

To verify that the tested combinations act synergistically against microorganisms of the natural microbiota of mammalian skin, a corresponding experiment was conducted with *Aspergillus brasiliensis,* a well-known pathogen on the skin. The synergistic activities of different alkanediol mixtures including a 1,2-alkanediol (substance A) and a 2,3-alkanediol (substance B) in ratios as specified in the following Table 5 were determined by measuring the MIC of the mixture as indicated in methods section.

In order to calculate the synergy index with MIC values, the Kull equation (I) was used as follows:

$$SI = (MIC_{mix} \times P_A) / MIC_A + (MIC_{mix} \times P_B) / MIC_B$$

wherein

SI is the Synergy Index according to Kull
$MIC_A$ is the MIC value for Substance A in ppm
$MIC_B$ is the MIC value for Substance B in ppm
$MIC_{mix}$ is the MIC value for the mixture of substances A and B and C in ppm
$P_A$ is the proportion of the substance A in the mixture
$P_B$ is the proportion of the substance B in the mixture

**Table 17:** Synergy index of different alkanediol mixtures.

| Comp. A | Comp. B | $P_A$ | $P_B$ | $MIC_A$ in [ppm] | $MIC_B$ [ppm] | $MIC_{mix}$ [ppm] | Synergy Index |
|---|---|---|---|---|---|---|---|
| 1,2-C6 | 2,3-C6 | 0.9 | 0.1 | 7500 | 20000 | 7500 | 0.94 |
| 1,2-C7 | 2,3-C7 | | | 5000 | 7500 | 2500 | 0.49 |
| 1,2-C8 | 2,3-C8 | | | 2500 | 2500 | 500 | 0.20 |
| 1,2-C9 | 2,3-C9 | | | 500 | 1000 | 250 | 0.48 |
| * Octanediol = C8<br>Heptanediol = C7 | | | | | | | |

In addition, a synergistic antimicrobial efficacy could be observed for an alkanediol mixture including 1,2-heptanediol and 2,3-heptanediol (in a ratio of 90 : 10) against Pseudomonas aeruginosa. The Synergy Index for said alkanediol mixture was 0.50.

Such compositions or mixtures are thus useful in efficient malodour management products, in products for topical applications and in products for masking insect alluring odours. Moreover, the blend of 1,2-nononanediol and 2,3-nonanediol (in a ratio of 95 : 5) also has an antimicrobial efficacy against different yeast (e.g. *Candida albicans* (CA) and fungi (e.g. *Aspergillus brasiliensis* (AB)). Furthermore, a blend of 1,2-nononanediol and 2,3-nonanediol (in a ratio of 90 : 10) also has an antimicrobial efficacy against yeast (e.g. Candida albicans (CA)).

**Example 12:** Sensory properties of topical compositions comprising the compositions according to the present invention:

For evaluating the sensory behaviour of the inventive composition in combination with different liquid lipophilic components, the oil components specified in the following Table 17 were blended with 1,2-heptanediol (1,2-C7) or 2,3-heptanediol (2,3-C7) or a mixture of 1,2-heptanediol and 2,3-heptanediol (in a ratio of 95 : 5) or 1,2-octanediol (1,2-C8) or 2,3-octanediol (2,3-C8) or a mixture of 1,2-octanediol and 2,3-octanediol (in a ratio of 95 : 5) in a ratio of 85 : 15 and assessed by untrained panellists (indicated with "w" in Table 17) in comparison to the liquid lipophilic component without addition of the specified alkanediols (placebo; indicated with "w/o" in Table 17). Based thereon the difference of samples with (w) and without (w/o) the alkanediols was calculated. A high difference (Δ) indicates a bigger positive influence of the corresponding alkanediol-additive(s). Analogous experiments were also performed using the corresponding hexanediol homologues.

Test procedure:

- Hands were pre-washed with liquid soap by following a defined procedure;
- 30 μl of corresponding sample was applied on the backside of each hand (randomized);
- After 30 s the spreading behaviour of the respective formulation on skin was evaluated versus the respective oil component without 1,2-heptanediol on a scale from 1 to 5 (1 = low spreading; 5 = high spreading);
- The samples were distributed with the fingertips of middle and forefinger with 2 x 5 cycles;
- After 2 minutes, the fatty/greasy skin feel was evaluated on a scale from 1 to 5 (1 = low fatty/greasy skin feel; 5 = high fatty/greasy skin feel);
- Furthermore, the applicants were asked to assess the absorption, i.e., the impression of amount of remaining residue on the skin on a scale from 1 to 5 (1 = low absorption; 5 = high absorption).

**Table 18:** Results of the sensory properties of topical compositions comprising the alkanediol-compositions according to the invention.

| | | Spreading | | | Fatty / greasy Skin Feel | | | Absorption | | |
|---|---|---|---|---|---|---|---|---|---|---|
| Lipid (plant oils) | Alkanediol | w/o | w | Δ | w/o | w | Δ | w/o | w | Δ |
| Argan Oil | 1,2-C8 | 2 | 3.8 | 1.8 | 4 | 2.6 | 1.4 | 3.8 | 2.6 | 1.2 |

(continued)

| | | Spreading | | | Fatty / greasy Skin Feel | | | Absorption | | |
|---|---|---|---|---|---|---|---|---|---|---|
| Lipid (plant oils) | Alkanediol | w/o | w | Δ | w/o | w | Δ | w/o | w | Δ |
| Argan Oil | 1,2-C8 / 2,3-C8 (95:5) | 2 | 4.2 | **2.2** | 4 | 2.2 | **1.8** | 3.8 | 2 | **1.8** |
| Argan Oil | 2,3-C8 | 2 | 3.4 | 1.4 | 4 | 2.4 | 1.6 | 3.8 | 2.2 | 1.6 |
| Avocado Oil | 1,2-C8 | 2 | 4 | 2 | 3.8 | 3 | 0.8 | 3.8 | 2.6 | 1.2 |
| Avocado Oil | 1,2-C8 / 2,3-C8 (95:5) | 2 | 3.8 | 1.8 | 3.8 | 2.2 | **1.6** | 3.8 | 2.2 | **1.6** |
| Avocado Oil | 2,3-C8 | 2 | 3.2 | 1.2 | 3.8 | 2.8 | 1 | 3.8 | 2.4 | 1.4 |
| Cacao butter | 1,2-C7 | 1.8 | 3.8 | 2 | 4.2 | 3 | 1.2 | 3.6 | 3.6 | 0 |
| Cacao butter | 1,2-C7/2,3-C7 (95:5) | 1.8 | 4 | **2.2** | 4.2 | 3 | **1.2** | 3.6 | 3 | **0.6** |
| Cacao butter | 2,3-C7 | 1.8 | 3 | 1.2 | 4.2 | 3.4 | 0.8 | 3.6 | 3.2 | 0.4 |
| CCT (neutral oil) | 1,2-C7 | 1.9 | 3.4 | 1.5 | 3.2 | 2.9 | 0.3 | 3.3 | 2.7 | 0.6 |
| CCT (neutral oil) | 1,2-C7 / 2,3-C7 (95:5) | 1.9 | 3.8 | **1.9** | 3.2 | 2.6 | **0.6** | 3.3 | 2.8 | 0.5 |
| CCT (neutral oil) | 2,3-C7 | 1.9 | 3.6 | 1.7 | 3.2 | 3.4 | -0.2 | 3.3 | 3 | 0.3 |
| CCT (neutral oil) | 1,2-C6 | 1.8 | 3.6 | 1.8 | 4.6 | 3.2 | 1.4 | 4.6 | 2.8 | 1.8 |
| CCT (neutral oil) | 1,2-C6/2,3-C6 (95:5) | 1.8 | 3.6 | **1.8** | 4.6 | 3.0 | **1.6** | 4.6 | 2.6 | **2.0** |
| CCT (neutral oil) | 2,3-C6 | 1.8 | 3.2 | 1.4 | 4.6 | 3.2 | 1.4 | 4.6 | 2.8 | 1.8 |
| Grapeseed Oil | 1,2-C7 | 2 | 3.8 | 1.8 | 4.2 | 2.2 | 2 | 2.6 | 2.8 | 0.2 |
| Grapeseed Oil | 1,2-C7/2,3-C7 (95:5) | 2 | 3.8 | **1.8** | 4.2 | 2.2 | **2** | 2.6 | 1.6 | **1** |
| Grapeseed Oil | 2,3-C7 | 2 | 3.6 | 1.6 | 4.2 | 2.6 | 1.6 | 3 | 2.2 | 0.8 |
| Grapeseed Oil | 1,2-C8 | 2 | 4 | 2 | 4.2 | 2.8 | 1.4 | 2.6 | 1.6 | 1 |
| Grapeseed Oil | 1,2-C8/2,3-C8 (95:5) | 2 | 3.8 | 1.8 | 4.2 | 2.2 | 2 | 2.3 | 1.4 | 0.9 |
| Grapeseed Oil | 2,3-C8 | 2 | 2.8 | 0.8 | 4.2 | 2.8 | 1.4 | 2.6 | 2.4 | 0.2 |
| Helianthus Annuus (Sunflower) Seed Oil | 1,2-C8 | 1.2 | 4.2 | 3 | 3.8 | 2 | 1.8 | 3.3 | 2 | 1.3 |
| Helianthus Annuus (Sunflower) Seed Oil | 1,2-C8/2,3-C8 (95:5) | 1.2 | 4.4 | **3.2** | 3.8 | 2.4 | 1.4 | 3.3 | 2.6 | 0.7 |
| Helianthus Annuus (Sunflower) Seed Oil | 2,3-C8 | 1.2 | 3.4 | 2.2 | 3.8 | 3 | 0.8 | 3.3 | 3.4 | -0.1 |
| Olive Oil | 1,2-C7 | 2.2 | 4 | 1.8 | 3.8 | 2 | 1.8 | 4 | 2.4 | 1.6 |
| Olive Oil | 1,2-C7 / 2,3-C7 (95:5) | 2.2 | 3.8 | 1.6 | 3.8 | 1.8 | **2** | 4 | 2 | **2** |
| Olive Oil | 2,3-C7 | 2.2 | 4 | 1.8 | 3.8 | 2.2 | 1.6 | 4 | 2 | 2 |
| Olive Oil | 1,2-C8 | 2.2 | 4.2 | 2 | 3.8 | 2.6 | 1.2 | 4 | 2.6 | 1.4 |
| Olive Oil | 1,2-C8/2,3-C8 (95:5) | 2.2 | 4.4 | **2.2** | 3.8 | 2.2 | **1.6** | 4 | 2.4 | **1.6** |

(continued)

| | | Spreading | | | Fatty / greasy Skin Feel | | | Absorption | | |
|---|---|---|---|---|---|---|---|---|---|---|
| **Lipid (plant oils)** | **Alkanediol** | **w/o** | **w** | **Δ** | **w/o** | **w** | **Δ** | **w/o** | **w** | **Δ** |
| Olive Oil | 2,3-C8 | 2.4 | 3.2 | 0.8 | 3.8 | 2.4 | 1.4 | 4 | 2.6 | 1.4 |
| **Fatty ester** | **Alkanediol** | **w/o** | **w** | **Δ** | **w/o** | **w** | **Δ** | **w/o** | **w** | **Δ** |
| Octocrylene | 1,2-C7 | 1.0 | 3.5 | 2.5 | 4.0 | 3.5 | 0.5 | 4.0 | 3.5 | 0.5 |
| Octocrylene | 1,2-C7 /2,3-C7 (95:5) | 1.0 | 4.0 | **3.0** | 4.0 | 3.0 | **1.0** | 4.0 | 2.5 | **1.5** |
| Octocrylene | 2,3-C7 | 1.0 | 3.5 | 2.5 | 4.0 | 3.0 | 1.0 | 4.0 | 3.5 | 0.5 |
| Octocrylene | 1,2-C8 | 1.0 | 3.5 | 2.5 | 4.0 | 3.5 | 0.5 | 4.0 | 3.5 | 0.5 |
| Octocrylene | 1,2-C8 / 2,3-C8 (95:5) | 1.0 | 4.5 | **3.5** | 4.0 | 2.5 | **1.5** | 4.0 | 2.5 | **1.5** |
| Octocrylene | 2,3-C8 | 1.0 | 3.5 | 2.5 | 4 | 3.0 | 1.0 | 4.0 | 2.5 | 1.5 |
| * Caprylic Capric Triglycerides = CCT<br>Octanediol = C8<br>Heptanediol = C7<br>Hexanediol = C6<br>Octocrylene = Octocrylene (Neo Heliopan 303) | | | | | | | | | | |

Higher delta values (Δ) indicate better spreading properties, less fatty/greasy skin feel and less remaining residue on skin/ better absorption, respectively.

It was found that the use of 1,2-heptanediol or 2,3-heptanediol or a mixture of 1,2-heptanediol and 2,3-heptanediol or 1,2-octanediol or 2,3-octanediol or a mixture of 1,2-octanediol and 2,3-octanediol or 1,2-hexanediol or 2,3-hexanediol or a mixture of 1,2-hexanediol and 2,3-hexanediol improves the spreading behaviour and/or significantly reduces the fatty/greasy skin feel of oily ingredients. In parallel, the absorption (less remaining residue) was likewise improved. In particular, it was found that the combination of 1,2-alkanediols with minor portions of 2,3-alkandiols is able to considerably improve the afore-mentioned properties for specific lipid compositions. Thereby, the improvement was more pronounced compared to the single alkanediol substances indicating a synergistic effect of said isomeric alkanediol mixtures.

## Claims

**1.** Process for the preparation of alkanediols having 5 to 15 carbon atoms comprising the following steps:

a) providing a 1-alcohol having 5 to 15 carbon atoms and/or a dialkylether having 10 to 30 carbon atoms and a catalyst;

b) dehydrating the 1-alcohol and/or cleavage of the dialkylether to obtain at least one alkene having 5 to 15 carbon atoms, wherein the at least one alkene having 5 to 15 carbon atoms is at least one olefin selected from the group consisting of: α-, β- and/or γ-olefins;

wherein the dehydration and/or cleavage is performed with recirculation of dialkylether and/or 1-alcohol; and wherein the dehydration and/or cleavage is performed at a temperature ranging from 255 °C to 290 °C;

c) reaction of the at least one alkene of step b) to obtain a product comprising or consisting of at least one alkanediol having 5 to 15 carbon atoms.

**2.** Process for the preparation of alkanediols having 5 to 15 carbon atoms according to claim 1, wherein in step c) a product is obtained comprising or consisting of at least two alkanediols selected from the group consisting of: a 1,2-alkanediol, a 2,3-alkanediol and a 3,4-alkanediol, each independently having 5 to 15 carbon atoms; wherein in the product, the ratio of the 1,2-alkanediol to the 2,3-alkanediol is in the range of 90 : 10 to 99.9 : 0.1 and/or the ratio

of the 1,2-alkanediol to the 3,4-alkanediol is in the range of 90 : 10 to 99.99 : 0.01 by weight.

**3.** Process according to any one of claims 1 or 2, wherein the 1-alcohol has 6 to 9 carbon atoms; or

wherein the catalyst is unmodified γ-alumina;
or
wherein the dehydration and/or cleavage of step b) is performed at a temperature ranging from 270 °C to 280 °C;
wherein the liquid hourly space velocity of the reactants over the catalyst within the reaction chamber is ranging from 0.5 to 10 1/h, preferably 1 to 5 1/h;
or
wherein the reaction chamber is a fixed bed reactor;
or
wherein dialkylether and/or 1-alcohol are recirculated and combined with fresh 1-alcohol and/or fresh dialkylether, preferably fresh 1-alcohol, in a ratio of 95 : 5 to 5 : 95, preferably in a ratio of 80 : 20 to 20 : 80 by weight;
or
wherein the dehydration step b) is performed as a continuous steady-state process; or
wherein the process additionally comprises at least one distillation step after step b) and/or step c);
or
wherein step c) is accomplished by reacting the at least one alkene with formic acid and peroxide followed by subsequent reaction with water and sodium hydroxide.

**4.** Composition comprising at least two alkanediols selected from the group consisting of: a 1,2-alkanediol, a 2,3-alkanediol and a 3,4-alkanediol, each independently having 5 to 15 carbon atoms;

wherein the ratio of the 1,2-alkanediol to the 2,3-alkanediol is in the range of 90 : 10 to 99.9 : 0.1 by weight and/or wherein the ratio of the 1,2-alkanediol to the 3,4-alkanediol is in the range of 90 : 10 to 99.99 : 0.01 by weight.

**5.** Composition according to claim 4, wherein the composition comprises a 1,2-alkanediol and a 2,3-alkanediol, each independently having 5 to 15 carbon atoms, and wherein the ratio of the 1,2-alkanediol to the 2,3-alkanediol is in the range of 90 : 10 to 99.9 : 0.1 by weight.

**6.** Composition according to claim 4, wherein the composition comprises a 1,2-alkanediol and a 3,4-alkanediol, each independently having 5 to 15 carbon atoms, and wherein the ratio of the 1,2-alkanediol to the 3,4-alkanediol is in the range of 90 : 10 to 99.99 : 0.01 by weight.

**7.** Composition according to claim 4, wherein the composition comprises a 1,2-alkanediol, a 2,3-alkanediol and a 3,4-alkanediol, each independently having 5 to 15 carbon atoms, wherein the ratio of the 1,2-alkanediol to the 2,3-alkanediol is in the range of 90 : 10 to 99.9 : 0.1, and wherein the ratio of the 1,2-alkanediol to the 3,4-alkanediol is in the range of 90 : 10 to 99.99 : 0.01 by weight.

**8.** Composition according to any one of claims 4 to 7, wherein the 1,2-alkanediol and the 2,3-alkanediol and/or the 1,2-alkanediol and the 3,4-alkanediol and/or the 2,3-alkanediol and the 3,4-alkanediol have the same chain lengths; or
wherein the 1,2-alkanediol and the 2,3-alkanediol have the same chain lengths.

**9.** Composition according to claim 8, wherein the alkanediols are alkanediols each independently having chain lengths ranging from 5 to 9 carbon atoms, and preferably from 7 to 8 carbon atoms.

**10.** Use of the composition according to any one of claims 4 to 9 for the preparation of consumer products such as cosmetics, personal care products, in particular skin cleaning products, shampoos, rinse-off conditioners, deodorants, antiperspirants, body lotions, homecare products, textile care products, household products, in particular liquid detergents, all-purpose cleaners, laundry and cleaning agents, fabric softeners, scent boosters, fragrance enhancers and pharmaceutical compositions.

**11.** Consumer products such as cosmetics, personal care products, in particular skin cleaning products, shampoos, rinse-off conditioners, deodorants, antiperspirants, body lotions, homecare products, textile care products, household products, in particular liquid detergents, all-purpose cleaners, laundry and cleaning agents, fabric softeners, scent

boosters, fragrance enhancers and pharmaceutical compositions comprising the composition according to any one of claims 4 to 9.

**12.** Process for the preparation of alkanediols having 5 to 15 carbon atoms comprising the following steps:

a) providing at least one 1-alcohol having 5 to 15 carbon atoms, preferably two or more 1-alcohols having 5 to 15 carbon atoms, and/or at least one dialkylether having 10 to 30 carbon atoms and a catalyst;
b) dehydrating the at least one 1-alcohol and/or cleavage of the at least one dialkylether to obtain at least one alkene having 5 to 15 carbon atoms, wherein the at least one alkene having 5 to 15 carbon atoms is at least one olefin selected from the group consisting of: α-, β- and/or γ-olefins, each independently having 5 to 15 carbon atoms;

wherein the dehydration and/or cleavage is performed with recirculation of dialkylether and/or 1-alcohol; and wherein the dehydration and/or cleavage is performed at a temperature ranging from 255 °C to 290 °C;

c) reaction of the at least one alkene of step b) to obtain a product comprising or consisting of at least one alkanediol having 5 to 15 carbon atoms.

**13.** Process according to claim 12, wherein in step c) a product is obtained comprising or consisting of at least two 1,2-alkanediols differing in their chain length and/or a product comprising or consisting of at least two 2,3-alkanediols differing in their chain length, wherein said alkanediols each independently have from 5 to 15 carbon atoms.

**14.** Composition comprising or consisting of at least two 1,2-alkanediols differing in their chain length and/or composition comprising or consisting of at least two 2,3-alkanediols differing in their chain length, wherein said alkanediols each independently have from 5 to 15 carbon atoms, obtained or obtainable by the process according to claim 12 or claim 13.

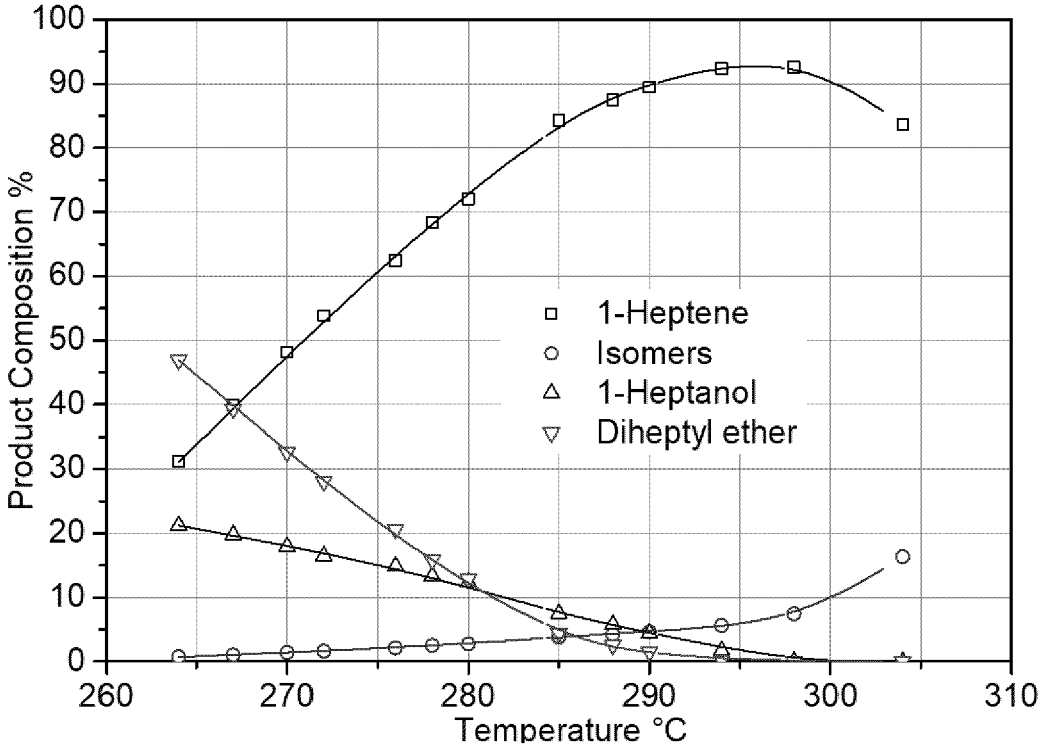


**Figure 1A**

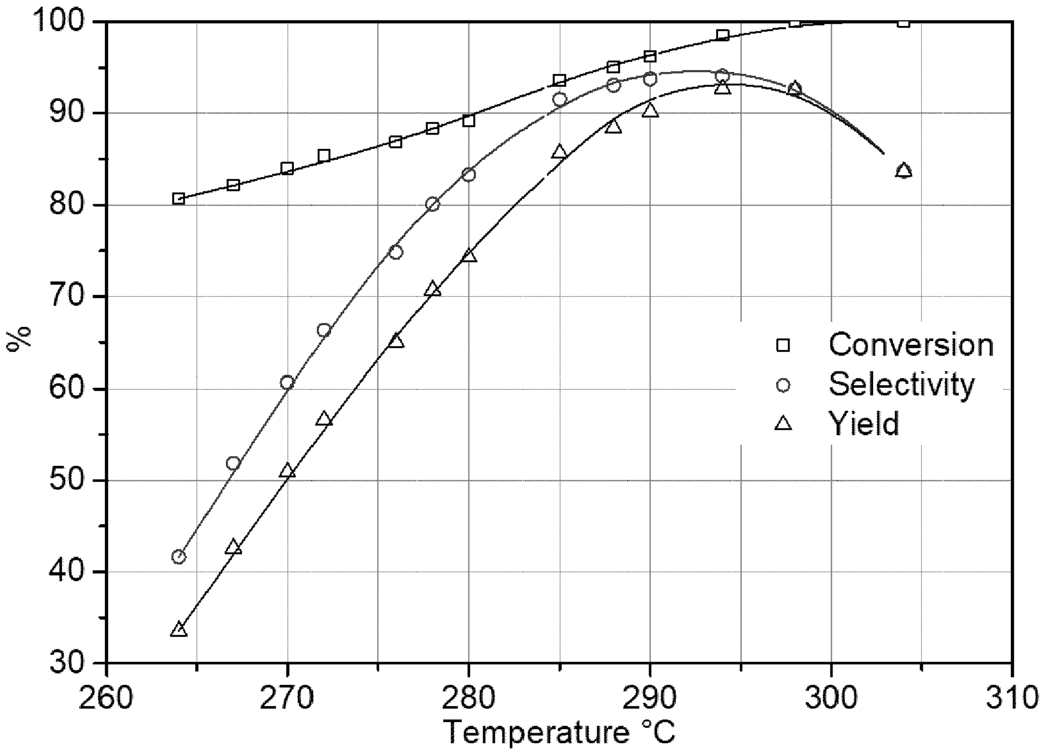


**Figure 1B**

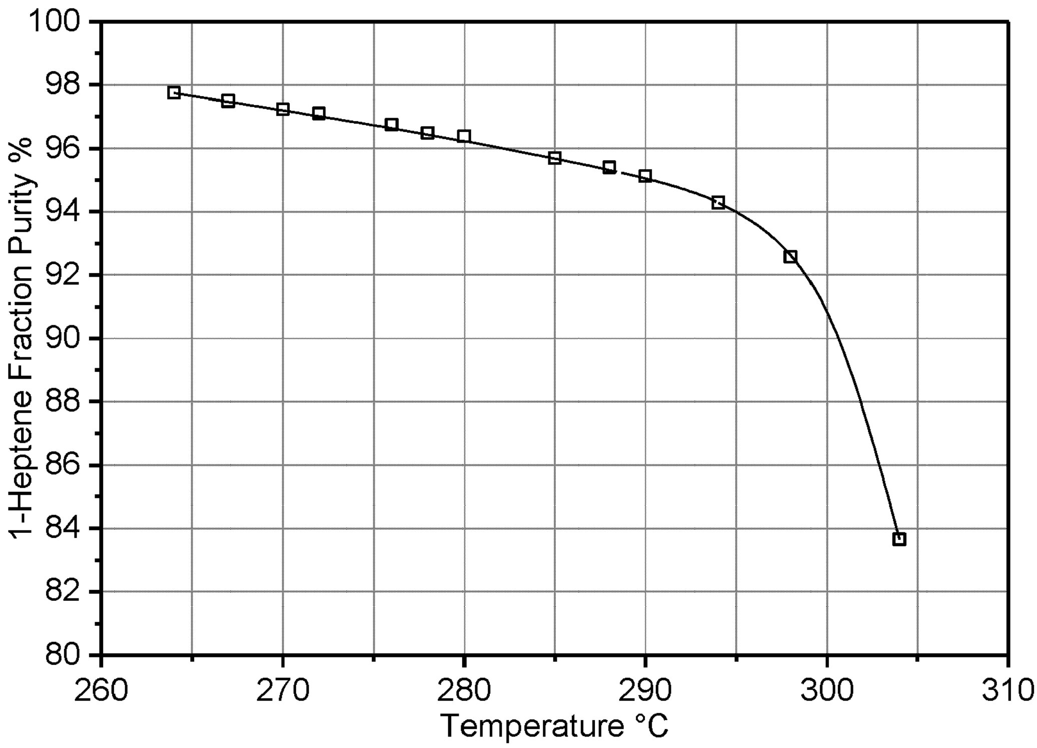
100
98
96
94
92
90
88
86
84
82
80
1-Heptene Fraction Purity %
260
270
280
290
300
310
Temperature °C

**Figure 1C**

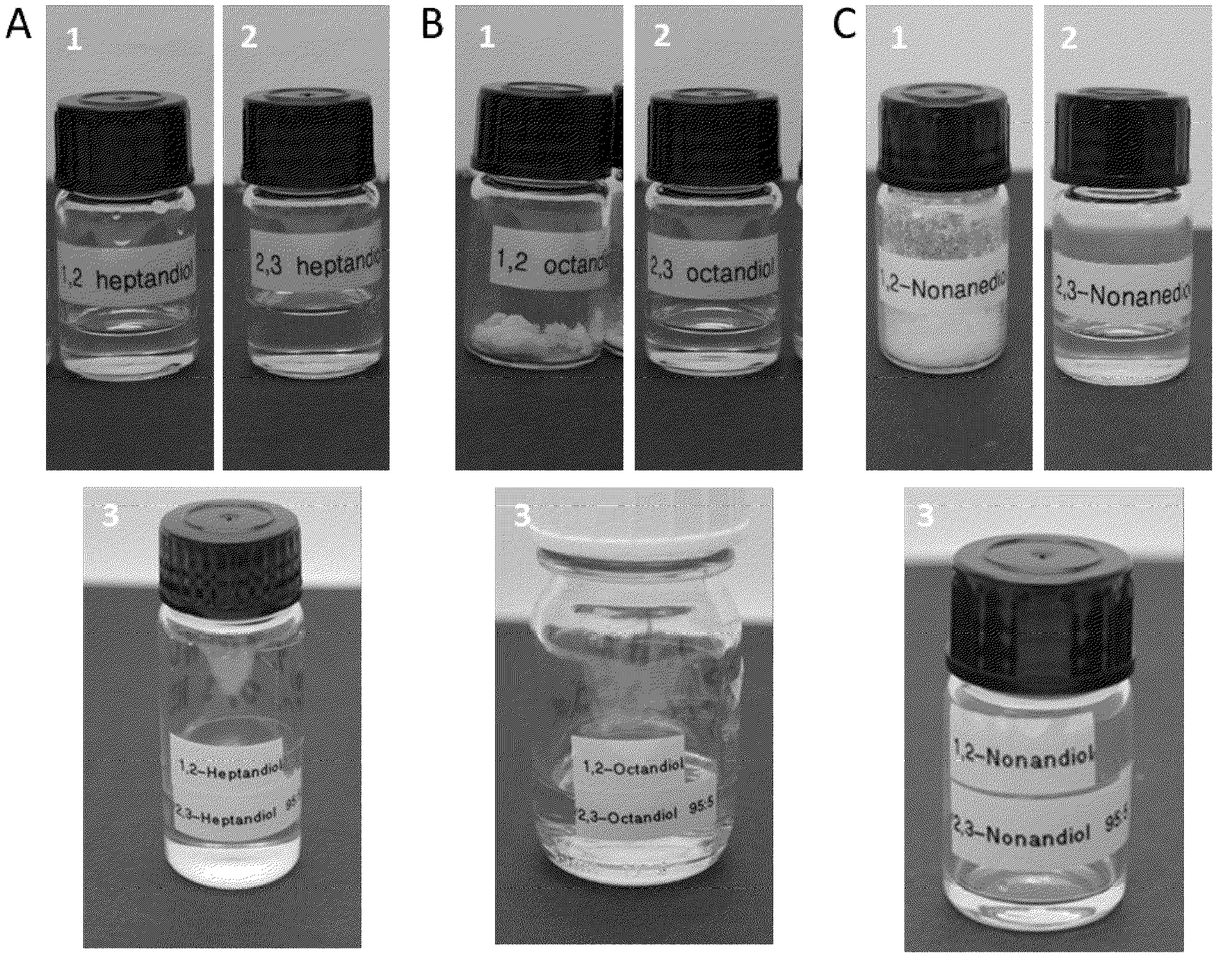


**Figures 2A to 2C**

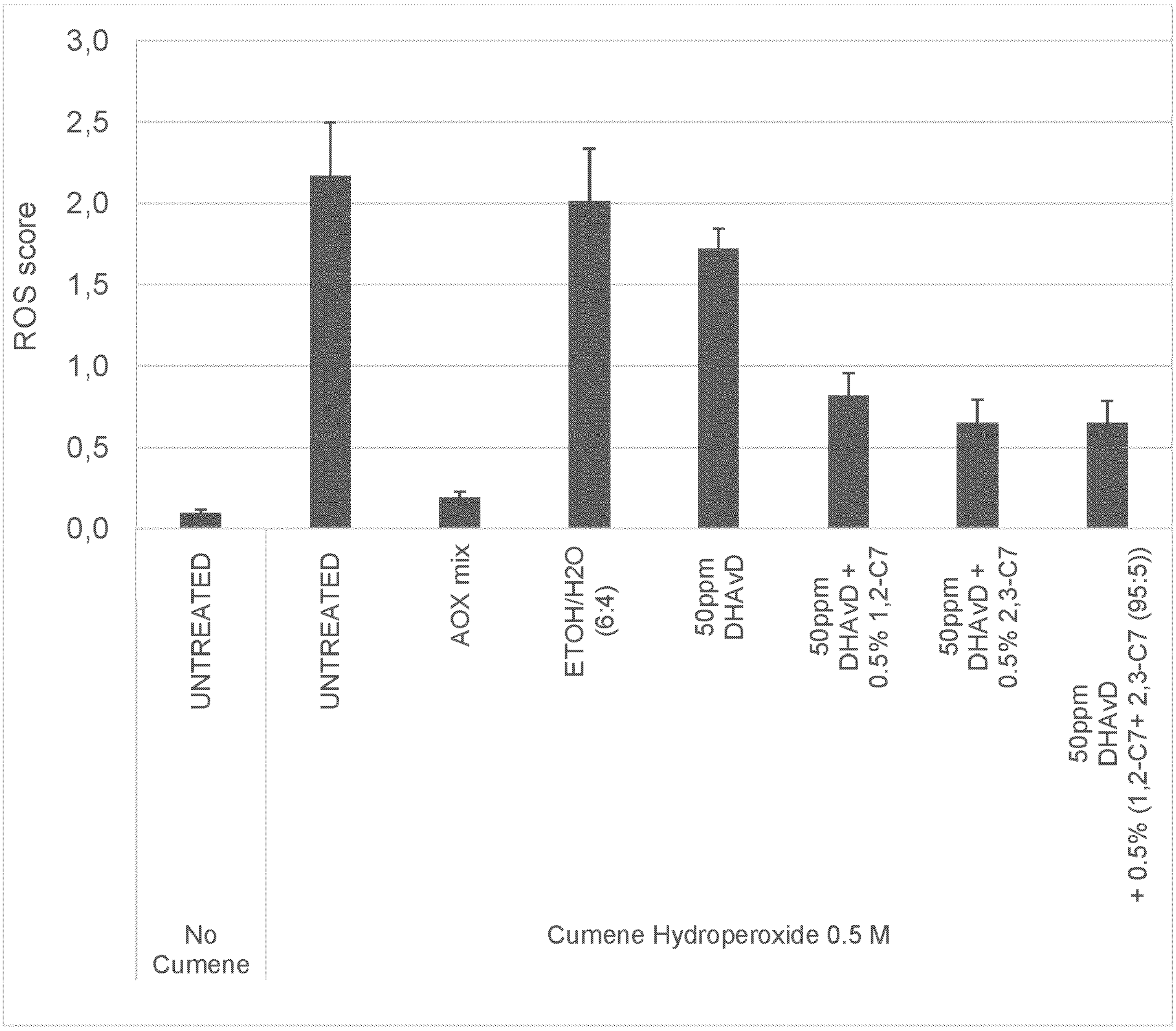
3,0
2,5
2,0
1,5
1,0
0,5
0,0
ROS score
UNTREATED
UNTREATED
AOX mix
ETOH/H2O (6:4)
50ppm DHAvD
50ppm DHAvD + 0.5% 1,2-C7
50ppm DHAvD + 0.5% 2,3-C7
50ppm DHAvD + 0.5% (1,2-C7+ 2,3-C7 (95:5))
No Cumene
Cumene Hydroperoxide 0.5 M

Figure 3

Europäisches Patentamt
European Patent Office
Office européen des brevets

# EUROPEAN SEARCH REPORT

Application Number
EP 24 18 6039

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br>A | WO 2019/152569 A2 (INOLEX INVESTMENT CORP [US]) 8 August 2019 (2019-08-08)<br>* claims 42-55 *<br>----- | 4-11,14<br>1-3,12,13 | INV.<br>C07C1/20<br>C07C1/24<br>C07C11/02 |
| X | WO 2019/029808 A1 (SYMRISE AG [DE]) 14 February 2019 (2019-02-14)<br>* claims 13,14; tables 8,10,11,43,45 *<br>----- | 4-11,14 | |
| X | WO 2006/069953 A1 (SYMRISE GMBH & CO KG [DE]; SCHMAUS GERHARD [DE]; PILLAI RAVIKUMAR [US]) 6 July 2006 (2006-07-06)<br>* Claims, examples *<br>----- | 4-11,14 | |

TECHNICAL FIELDS SEARCHED (IPC)

C07C

The present search report has been drawn up for all claims

2

| Place of search | Date of completion of the search | Examiner |
|---|---|---|
| The Hague | 4 November 2024 | Fritz, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 18 6039

This annex lists the patent family members relating to the patent documents cited in the above-mentioned European search report. The members are as contained in the European Patent Office EDP file on
The European Patent Office is in no way liable for these particulars which are merely given for the purpose of information.

04-11-2024

| Patent document cited in search report | Publication date | Patent family member(s) | Publication date |
|---|---|---|---|
| WO 2019152569 A2 | 08-08-2019 | AU 2019214975 A1 | 07-01-2021 |
| | | CA 3097110 A1 | 08-08-2019 |
| | | CN 115362141 A | 18-11-2022 |
| | | EP 3758671 A2 | 06-01-2021 |
| | | JP 2021519816 A | 12-08-2021 |
| | | JP 2024062989 A | 10-05-2024 |
| | | US 2019241491 A1 | 08-08-2019 |
| | | US 2019289848 A1 | 26-09-2019 |
| | | US 2020189995 A1 | 18-06-2020 |
| | | US 2022402844 A1 | 22-12-2022 |
| | | WO 2019152569 A2 | 08-08-2019 |
| WO 2019029808 A1 | 14-02-2019 | CN 109384650 A | 26-02-2019 |
| | | EP 3664772 A1 | 17-06-2020 |
| | | EP 4331684 A2 | 06-03-2024 |
| | | JP 7122814 B2 | 22-08-2022 |
| | | JP 2019055931 A | 11-04-2019 |
| | | KR 20190016877 A | 19-02-2019 |
| | | KR 20190086639 A | 23-07-2019 |
| | | US 2021085579 A1 | 25-03-2021 |
| | | US 2023355491 A1 | 09-11-2023 |
| | | WO 2019029808 A1 | 14-02-2019 |
| WO 2006069953 A1 | 06-07-2006 | EP 1835843 A1 | 26-09-2007 |
| | | JP 2008525518 A | 17-07-2008 |
| | | US 2010216892 A1 | 26-08-2010 |
| | | WO 2006069953 A1 | 06-07-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1876162 A1
- US 6528665 B1
- EP 0257243 A1
- EP 0141775 A1
- WO 2019152569 A2